# EUROPEAN PATENT APPLICATION

(11) **EP 0 945 446 A1**
(43) Date of publication of application: **29.09.1999**
(21) Application number: 96938465.0
(22) Date of filing: 14.11.1996
(51) Int. Cl.: C07D 251/70

(54) **CYANOETHYLMELAMINE DERIVATIVES AND PROCESS FOR PRODUCING THE SAME**

(71) Applicant: NISSAN CHEMICAL INDUSTRIES, LIMITED, Chiyoda-ku Tokyo 101 (JP)
(72) Inventor: TANAKA, Norio, Nissan Chemical Ind., Ltd. Central, Funabashi-shi, Chiba-ken 274-8507 (JP); MIZUSAWA, Kenichi, Nissan Chemical Ind., Ltd., Funabashi-shi, Chiba-ken 274-8507 (JP); ISHIKAWA, Makoto, Nissan Chemical Ind., Ltd., Funabashi-shi, Chiba-ken 274-8507 (JP); FUKUE, Yasuo, Nissan Chemical Ind., Ltd., Funabashi, Chiba-ken 274-8507 (JP); HASHIBA, Isao, Nissan Chemical Ind., Ltd., Funabashi-shi, Chiba-ken 274-8507 (JP)
(74) Representative: HOFFMANN - EITLE
(86) International application number: JP9603340
(87) International publication number: WO9821191

(57) **Abstract**

The present invention relates to a cyanoethylmelamine derivatives of the formula I: 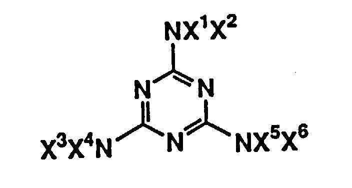 {wherein 1 to 5 of the substituents X' to X⁶ represent a cyanoethyl group and at least one remaining group represents a C₁₋₂₀ alkyl group, a C₂₋₂₀ alkenyl group (said alkyl or alkenyl group may optionally have an alicyclic group or a phenyl group in its structure), a C₅₋₆ cycloalkyl group or a phenyl group, or two of the substituent groups bonded to the same nitrogen atom together may form an alkylene chain having 2 to 7 carbon atoms, and the remaining substituent or substituents represents a hydrogen atom}. The production of cyanoethylmelamine derivatives can be obtained at high yield by reacting N-substituted melamine derivatives with acrylonitrile or reacting mono- or di-halogeno-N-substituted triazine derivative with aminopropionitrile. The compounds of the formula (I) are useful compounds as intermediates for fine chemicals such as agricultural chemicals, medicines, dye stuffs, paints, etc. and as crosslinking intermediates for resins.

## Description

### TECHNICAL FIELD

The present invention relates to novel melamine derivatives useful as raw materials and intermediates for various fine chemicals such as agricultural chemicals, medicines, dye stuffs, paints and resins and to a process for producing the same.

### BACKGROUND ART

Heretofore, various functional group(s)-substituted heterocyclic compounds have been utilized as raw materials and intermediates for various fine chemicals such as agricultural chemicals, medicines, dye stuffs, paints, and resins.

Among them, triazine derivatives having a substituted amino group are utilized for a number of applications not to speak of medical and agricultural chemicals and resin materials. In particular, 2,4,6-triamino-1,3,5-triazines, typically melamine, have a large number of functional groups and strength in skeleton so that they have been utilized as a crosslinking agent for amino resins such as melamine resins which has a very high crosslinking density in structure and various general-purpose resins.

On the other hand, melamine is not always so highly reactive and therefore it is only practically easy to react it with formaldehyde, so that it is used mainly as a crosslinking agent achieving a very short inter-crosslinking-site distance as in the above-mentioned applications. From this fact, it follows that resins obtained by using melamine directly as a crosslinking agent have limited freedom in the vicinity of crosslinking points so that although they have sufficient hardness, heat resistance and the like, they have defects that they are inferior in impact resistance and processability. Because of their reaction type, their use has been almost exclusively limited to amino resins.

As derivatives having a similar structure, it was described that a carboxylic acid derivative obtained by synthesizing a cyanoethyl derivative of benzoguanamine shown below and then hydrolyzing it is a useful intermediate for the synthesis of alkyd resins and plastics (U.S. Patent No. 2,577,477).

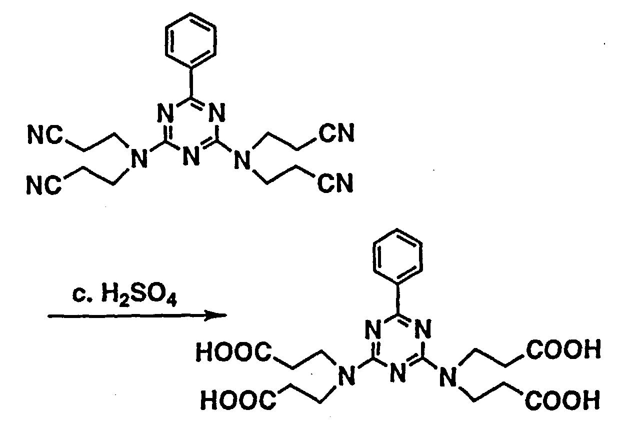

Also, various derivatives having a cyanoethyl group have been synthesized and studied as a physiologically active substance. In J. Med. Chem. vol. 36, p.4195 (1993) a compound having the following chemical structure is described as a melamine derivative having carcinostatic effects.

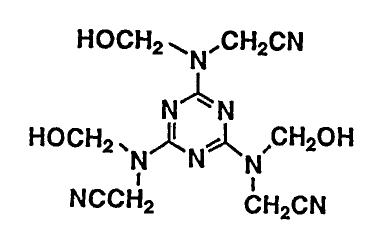

In order to solve the above-mentioned problems, the present inventors have made intensive research for finding novel melamine derivatives as novel polyfunctional intermediates which maintain properties required for crosslinking agents, such as heat resistance and polyfunctionality, as in the case of conventional melamine and which further have a different inter-crosslinking-site distance compared with conventional one. As a result, cyanoethylmelamine derivatives, novel polyfunctional compounds have been established, which can be obtained by reaction of substituted melamines which are raw materials available at low costs industrially, with acrylonitrile or by reaction of halogenated N-substituted triazines which are easily obtained by reacting cyanuric halides with amines, with aminopropionitrile and which is useful as a polyfunctional crosslinking intermediate and also useful as an intermediate for other fine chemical raw materials and as an intermediate for physiologically active substances such as medicinal, and agricultural chemicals and a method for producing the same has been established, thus completing the present invention.

An object of the present invention is to provide cyanoethylmelamine derivatives which are novel polyfunctional compounds and can be obtained by reaction of substituted melamines, which are available at low costs also industrially, with acrylonitrile or by reaction of halogenated N-substituted triazines, which are easily obtained by reacting cyanuric halides with amines, with aminopropionitrile and which is useful as a polyfunctional crosslinking intermediate and also useful as an intermediate for other fine chemical raw materials and as an intermediate for physiologically active substances such as medicinal and agricultural chemicals and a method for producing the same.

### DISCLOSURE OF THE INVENTION

That is, the present invention relates to a cyanoethylmelamine derivative that can be obtained by reaction of substituted melamines, which are available at low costs industrially, with acrylonitrile or by reaction of halogenated N-substituted triazines, which are easily obtained by reacting cyanuric halides with amines, with aminopropionitrile and to a method for producing the same. The cyanoethylmelamine derivative is a novel polyfunctional compound that is useful as a polyfunctional crosslinking intermediate and also useful as an intermediate for other fine chemical raw materials and as an intermediate for physiologically active substances such as medicinal and agricultural chemicals. Hereafter, the present invention will be described in more detail. The cyanoethylmelamine derivative of the present invention is the following melamine derivative, i.e., the melamine derivative of the formula I: 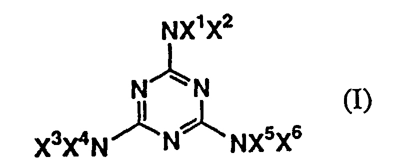 {wherein X¹, X², X³, X⁴, X⁵ and X⁶ independently represent respective substituents, 1 to 5 of which represent a cyanoethyl group and at least one remaining group represents a C₁₋₂₀ alkyl group, a C₂₋₂₀ alkenyl group (said alkyl or alkenyl group may optionally have an alicyclic group or a phenyl group in its structure), a C₅₋₆ cycloalkyl group or a phenyl group, or two of the substituent groups bonded to the same nitrogen atom together may form an alkylene chain having 2 to 7 carbon atoms, and the remaining substituent or substituents represents a hydrogen atom}.

Also, the method for producing the cyanoethylmelamine derivative of the present invention includes two production methods, i.e.,
A) a method for obtaining a cyanoethylmelamine derivative of the formula I (wherein the definitions of X¹, X², X³, X⁴, X⁵ and X⁶ are the same as in claim 1) described in claim 1 by reacting an N-substituted melamine derivative of the formula II: 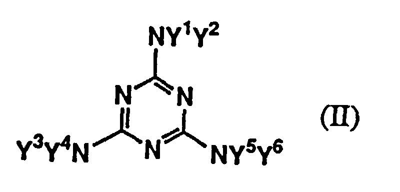 {wherein Y¹, Y², Y³, Y⁴, Y⁵ and Y⁶ independently represent respective substituents, 1 to 5 of which represent a C₁₋₂₀ alkyl group, a C₂₋₂₀ alkenyl group (said alkyl or alkenyl group may optionally have an alicyclic group or a phenyl group in its structure), a C₅₋₆ cycloalkyl group or a phenyl group, or two of the substituents bonded to the same nitrogen atom together may form an alkylene chain having 2 to 7 carbon atoms, and the remaining 5 to 1 substituents represent a hydrogen atom) with acrylonitrile, and
B) a method for producing a cyanoethylmelamine derivative of the formula I (wherein the definitions of X¹, X², X³, X⁴, X⁵ and X⁶ are the same as in claim 1, provided that at least one of X¹ to X⁶ bonded to the nitrogen atoms to which the cyanoethyl group is bonded is a hydrogen atom) described in claim 1 by reacting an N-substituted triazine derivative of the formula III: 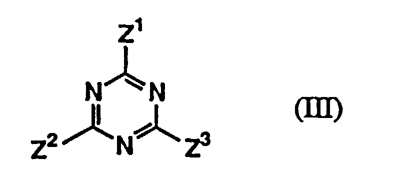 [ wherein Z¹, Z² and Z³ independently represent respective substituents, 1 or 2 of which independently represent NX⁷X⁸ group (wherein X⁷ or X⁸ independently represents a C₁₋₂₀ alkyl group, a C₂₋₂₀ alkenyl group (said alkyl or alkenyl group may optionally have an alicyclic group or a phenyl group in its structure), a C₅₋₆ cycloalkyl group, a phenyl group or a hydrogen atom, or X⁷ and X⁸ together may form an alkylene group having 2 to 7 carbon atoms), and the remaining 2 or 1 substituents represent a halogen atom] with aminopropionitrile.

Hereafter, the production method of the present invention will be explained.

The method shown in production method A) is a method for producing a cyanoethylmelamine derivative by Michael addition reaction in which acrylonitrile is added to various N-substituted melamine derivatives. In this reaction, a cyanoethyl group can be introduced to a portion of or whole NH groups of N-substituted melamine derivative as a raw material in a high yield.

The amount of acrylonitrile used in the reaction may vary depending on the number of cyanoethyl groups in the resulting cyanoethylmelamine derivative, the reactivity of N-substituted melamine derivative as a raw material, etc., but it is preferred to use it in the range of usually 0.5 to 50 moles, and 1.0 to 20 moles taking into consideration practical factors such as operativity, per mole of N-substituted melamine derivative as a raw material.

Although only the acrylonitrile reacts by addition with an N-substituted melamine derivative, it is desirable that a catalyst which activates the reaction is added in order to obtain a practically effective reaction rate.

The catalyst used in the reaction, which is general and highly used in industrial applications, includes alkali metal hydroxides such as sodium hydroxide, potassium hydroxide and lithium hydroxide, alkaline earth metal hydroxides such as barium hydroxide, strontium hydroxide, calcium hydroxide and magnesium hydroxide, alkali metal carbonates such as potassium carbonate, sodium carbonate and lithium carbonate, alkali metal hydrogencarbonates such as potassium hydrogencarbonate, sodium hydrogencarbonate and lithium hydrogencarbonate, alkaline earth metal carbonates such as barium carbonate, strontium carbonate, calcium carbonate and magnesium carbonate, organic amines such as diethylamine, triethylamine, tri-n-propylamine, tri-iso-propylamine, tri-n-butylamine, tri-n-hexylamine, tri-n-octylamine, diazabicycloundecene (DBU), pyridine, α-picoline, β-picoline, γ-picoline, methylethylpyridine, collidine, N,N-diemthylaniline, piperidine, morpholine, N-methylpiperidine and N-methylmorpholine, alkali metal alcoholates such as sodium methylate, sodium ethylate and potassium tert-butoxide, and various basic compounds such as benzyltrimethylammonium hydroxide, tetramethylammonium hydroxide and sodium amide. In addition, the catalyst for activating a substrate includes metal salts, complexes and other various substances such as cupric acetate, cuprous chloride, cuprous bromide, dihydridotetrakis(triphenylphosphine)ruthenium, dichlorotris( triphenylphosphine )ruthenium, trirutheniumdodecacarbonyl, palladium chloride, tetrakis(triphenylphosphine)palladium and dichlorobis(triphenylphosphine)palladium, complexes, etc.

The amount of catalyst to be added may vary depending on the reactivity of N-substituted melamine derivative as a raw material, etc., but preferably it is used usually in the range of 0.001 to 100 mole% and taking into consideration practical factors such as operativity, in the range of 0.005 to 50 moles, per mole of a raw material N-substituted melamine derivative.

In the reaction, the acrylonitrile as a raw material may be used as a solvent, but it is possible to use a solvent in order to improve operativity or for other purposes. The solvent which can be used in the reaction may be any so far as it does not participate in the reaction directly. Examples of the solvents are ethers such as tetrahydrofuran, diethyl ether, diethylene glycol diethyl ether and 1,4-dioxane, aromatic hydrocarbons such as benzene, toluene, xylene, mesitylene, cumene, chlorobenzene, o-dichlorobenzene, m-dichlorobenzene, p-dichlorobenzene and tetrahydronaphthalene, aliphatic hydrocarbons such as n-hexane, cyclohexane, n-pentane, n-octane and n-decane, nitriles such as acetonitrile, propionitrile and butyronitrile, alcohols such as methanol, ethanol, normalpropanol, isopropanol, normalbutanol, isobutanol, 2-methyl-2-propanol, normalamyl alcohol, normalhexanol, octanol and benzyl alcohol, amides such as N,N-dimethylformamide, N,N-dimethylacetamide and N-methylpyrrolidone, 1,3-dimethylimidazolidinone, ureas such as N,N,N',N'-tetramethylurea or water. These may be used singly or in combination.

The reaction temperature of the reaction may be generally in the range of 0 to 200°C . It is desirable that the reaction proceeds at a temperature usually in the range of from room temperature to 150 °C . When no solvent is used, it is desirable within the range of 77°C which is the boiling point of acrylonitrile.

The reaction time of the reaction depends on the reactivity of N-substituted melamine derivative to be used, but it is desirable that the reaction is completed usually in 5 minutes to 20 hours, preferably 10 minutes to 5 hours.

The reaction shown in production method B) is a production method in which a mono- or di-halogeno-N-substituted triazine derivative, which is easily derived from a cyanuric halide in one or two steps, is reacted with aminopropionitrile (cyanoethylamine) to obtain a cyanoethylmelamine derivative.

In this reaction, aminopropionitrile reacts with the raw material mono- or di-halogeno-N-substituted triazine derivative and a mono- or di-cyanoethylmelamine derivative is obtained in a good yield. Upon the reaction, aminopropionitrile is used 0.1 to 20 times moles, preferably 0.5 to 10 times moles, per mole of mono- or di-halogeno-N-substituted triazine derivative to synthesize corresponding cyanoethyl melamine derivative.

In the reaction, since a stoichiometric amount of hydrogen halide is by-produced, it is desirable that an equivalent amount of a base is added to the reaction system. Examples of the base which is used include alkali metal hydroxides such as sodium hydroxide, potassium hydroxide and lithium hydroxide, alkaline earth metal hydroxides such as barium hydroxide, strontium hydroxide, calcium hydroxide and magnesium hydroxide, alkali metal carbonates such as potassium carbonate, sodium carbonate and lithium carbonate, alkali hydrogencarbonates such as potassium hydrogencarbonate, sodium hydrogencarbonate and lithium hydrogencarbonate, alkaline earth metal carbonates such as barium carbonate, strontium carbonate, calcium carbonate and magnesium carbonate, organic amines such as diethylamine, triethylamine, tri-n-propylamine, tri-iso-propylamine, tri-n-butylamine, tri-n-hexylamine, tri-n-octylamine, diazabicycloundecene (DBU), pyridine, α-picoline, β-picoline, γ-picoline, methylethylpyridine, collidine, N,N-dimethylaniline, piperidine, morpholine, N-methylpiperidine and N-methylmorpholine. It is possible to use aminopropionitrile which is a reactant as a dehydrohalogenating agent, in an excess amount.

As for the amount of the above-mentioned dehydrohalogenating agent, it is desirable that the reaction is carried out with 0.1 to 5 times moles, preferably 0.5 to 3 times moles, per mole of the mono- or di-halogeno-N-substituted triazine derivative.

The reaction can proceed without solvents, but it is desirable to use solvents because of operativity, etc. Solvents are not limited particularly so far as they are inert to the reaction. Examples of the solvents are ethers such as tetrahydrofuran, diethyl ether, diethylene glycol diethyl ether and 1,4-dioxane, aromatic hydrocarbons such as benzene, toluene, xylene, mesitylene, cumene, chlorobenzene, o-dichlorobenzene, m-dichlorobenzene, p-dichlorobenzene and tetrahydronaphthalene, aliphatic hydrocarbons such as n-hexane, cyclohexane, n-pentane, n-octane and n-decane, halogenated hydrocarbons such as methylene chloride, chloroform and dichloroethane, nitriles such as acetonitrile, propionitrile and butyronitrile, alcohols such as methanol, ethanol, normalpropanol, isopropanol, normalbutanol, isobutanol, 2-methyl-2-propanol, normalamyl alcohol, normalhexanol, octanol and benzyl alcohol, amides such as N,N-dimethylformamide, N,N-dimethylacetamide and N-methylpyrrolidone, 1,3-dimethylimidazolidinone, ureas such as N,N,N',N'-tetramethylurea and water. These may be used singly or in combination. An excess amount of aminopropionitrile may be used as a solvent.

The reaction temperature of the reaction may vary depending on the reactivity of a raw material mono- or di-halogeno-N-substituted triazine derivative, but the reaction is possible at a temperature generally in the range of 0 to 200°C . Usually, it is desirable to carry out the reaction at a temperature in the range of from room temperature to 150°C , preferably from 40°C to 120 °C . Also, the reaction time of the reaction depends on the reactivity of the halogeno-N-substituted melamine derivative to be used, but usually it is desirable that the reaction is completed in 5 minutes to 20 hours, preferably 10 minutes to 5 hours.

The raw material N-substituted melamine derivative in production method A) and raw material N-substituted triazine derivative in production method B) as described above can be easily synthesized from melamine or cyanuric chloride as raw materials. The methods for synthesizing them are described in detail in "s-triazines and derivatives," from the series "The Chemistry of Heterocyclic Compounds," E. M. Smolin and L. Rapoport, Interscience Publishers Inc., New York, 1959.

As a series of cyanoethylmelamine derivatives and a raw material N-substituted melamine derivative of the formula II or N-substituted triazine derivative of the formula III, derivatives having various substituents are raised. Examples of substituents represented by the C₁₋₂₀ alkyl group, C₂₋₂₀ alkenyl group (said alkyl or alkenyl group may optionally have an alicyclic group or a phenyl group in its structure), C₅₋₆ cycloalkyl group and phenyl group among the substituents Y¹ to Y⁶ bonded to the N-atom in the N-substituted melamine derivative of the formula II serving as a raw material for use in the production method A), X⁷ or X⁸ in NX⁷X⁸ of Z¹, Z² and Z³ of the N-substituted triazine derivative of the formula III serving as a raw material for use in the production method B) and the substituents X¹ to X⁶ on the N-atom in the cyanoethylmelamine derivative of the formula I include a methyl group, an ethyl group, an n-propyl group, an iso-propyl group, an n-butyl group, an iso-butyl group, a sec-butyl group, a tert-butyl group, a cyclopropylmethyl group, an n-pentyl group, an iso-pentyl group, a sec-pentyl group, a tert-pentyl group, a cyclopentyl group, an n-hexyl group, a cyclohexyl group, a cyclohexylmethyl group, a 4-methylcyclohexylmethyl group, an n-octyl group, a 2-ethylhexyl group, an n-nonyl group, an n-decyl group, an n-dodecyl group, an n-hexadecyl group, an n-octadecyl group, a benzyl group, a 1-phenethyl group, a 2-phenytyl group, 1-phenylpropyl group, a 3-phenylpropyl group, a vinyl group, an allyl group, a methallyl group, a crotyl group, a 2-pentenyl group, a 3-hexenyl group, a styryl group, a phenyl group, etc.; and
when two of the above-mentioned substituents on the same nitrogen atom together may form an alkylene chain having 2 to 7 carbon atoms, an aziridine ring, an azetidine ring, a pyrrolidine ring and a piperidine ring which are formed together with the nitrogen atom are raised. These substituents are merely exemplary and the present invention should not be limited thereto.

Treatment methods after completion of the whole reaction include optional removal of solvents by distillation, etc. or extraction of the product as a two phase system of water-organic solvent, and subsequent purification and isolation of the reaction product by recrystallization, distillation, chromatographic separation, etc., if need.

As described above, in the present invention, various compounds can be used as the raw material N-substituted melamine derivative and halogenated N-substituted triazine derivative and selection of production method results in selection of the number of cyanoethyl group to be introduced into the product so that various representative products with such combinations can be obtained.

Next, preferred embodiments of the present invention will be described.

Of the compounds of the present invention, the following are preferred cyanoethylmelamine derivatives of the formula I in which X¹, X², X³, X⁴, X⁵ and X⁶ independently represent respective substituents, 1 to 5 of which represent a cyanoethyl group, at least one remaining substituent represents a C₁₋₁₀ alkyl group, a cyclohexylmethyl group, a cyclohexyl group or a benzyl group, or two of the above-mentioned substituents when bonded to the same nitrogen atom together may form an alkylene chain having 4 or 5 carbon atoms, and the remaining substituent or substituents represent a hydrogen atom.

As for the production method A) of the present invention, a preferred production method is that
in the formula II, Y¹, Y², Y³, Y⁴, Y⁵ and Y⁶ independently represent respective substituents, 1 to 5 of which represent a C₁₋₁₀ alkyl group, a cyclohexylmethyl group, a cyclohexyl group or a benzyl group, or two of the above-mentioned substituents when bonded to the same nitrogen atom together may form an alkylene chain having 4 or 5 carbon atoms, and the remaining 5 to 1 substituents represent a hydrogen atom; and
in the formula I, X¹, X², X³, X⁴, X⁵ and X⁶ independently represent respective substituents, 1 to 5 of which represent a cyanoethyl group, at least one remaining substituent represents a C₁₋₁₀ alkyl group, a cyclohexylmethyl group, a cyclohexyl group or a benzyl group, or two of the above-mentioned substituents when bonded to the same nitrogen atom together may form an alkylene chain having 4 or 5 carbon atoms, and the remaining substituents represent a hydrogen atom.

As for the production method B), a preferred production method is that
in the formula III, Z¹, Z² and Z³ independently represents respective substituents, 1 or 2 of which independently represent an NX⁷X⁸ group (wherein X⁷ or X⁸ independently represents a C₁₋₄ alkyl group, a cycloalkyl group, or a hydrogen atom, or X⁷ and X⁸ together may form an alkylene chain having 4 or 5 carbon atoms), and the 2 or 1 remaining substituents represent a chlorine atom, and
in the formula I, X¹, X², X³, X⁴, X⁵ and X⁶ independently represent respective substituents, one or two of substituted amino groups NX¹X², NX³X⁴ and NX⁵X⁶ independently represent NX⁷X⁸ group (wherein X⁷ or X⁸ independently represents a C₁₋₄ alkyl group, a cyclohexylmethyl group or a hydrogen atom, or X⁷ and X⁸ together may form an alkylene chain having 4 or 5 carbon atoms), and the remaining 2 or 1 substituted amino groups represent a cyanoethylamino group.

Table 1 below shows main examples of the cyanoethylmelamine derivatives of the formula I obtained by the production method of the present invention, by combinations of the substituents. In the abbreviations of substituents in the table have the following meanings.

| | |
|---|---|
| Me: methyl group | Et: ethyl group |
| Pr: normalpropyl group | ME: isopropyl group |
| Bu: normalbutyl group | IB: isobutyl group |
| MP: secondary butyl group | TB: tertiary butyl group |
| PE: normalpentyl group | HE: normalhexyl group |
| EH: 2-ethylhexyl | Oc: normaloctyl group |
| AL: allyl group | ST: styryl group |
| Cy: cyclohexyl group | CM: cyclohexylmethyl group |
| Ph: phenyl group | Bz: benzyl group |
| CE: cyanoethyl group | |

Further, the -(CH₂)₄- group or -(CH₂)₅- group indicates that the nitrogen atom on the triazine ring forms a pyrrolidine ring or a piperidine ring, respectively, containing 4 or 5 methylene groups, respectively.

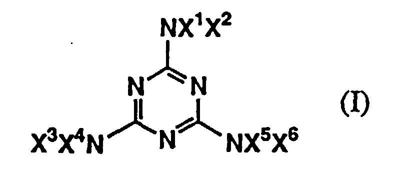

**TABLE 1-1**

| X¹ | X² | X³ | X⁴ | X⁵ | X⁶ |
|---|---|---|---|---|---|
| Me | H | H | H | H | CE |
| Me | CE | H | H | H | H |
| Me | H | H | H | CE | CE |
| Me | H | H | CE | H | CE |
| Me | CE | H | H | H | CE |
| Me | H | H | CE | CE | CE |
| Me | CE | H | H | CE | CE |
| Me | CE | H | CE | H | CE |
| Me | H | CE | CE | CE | CE |
| Me | CE | H | CE | CE | CE |
| Me | CE | CE | CE | CE | CE |
| Me | Me | H | H | H | CE |
| Me | Me | H | H | CE | CE |
| Me | Me | H | CE | H | CE |
| Me | Me | H | CE | CE | CE |
| Me | Me | CE | CE | CE | CE |
| Me | H | Me | H | H | CE |
| Me | H | Me | CE | H | H |
| Me | H | Me | H | CE | CE |
| Me | H | Me | CE | H | CE |
| Me | CE | Me | CE | H | H |
| Me | H | Me | CE | CE | CE |

**TABLE 1-2**

| X¹ | X² | X³ | X⁴ | X⁵ | X⁶ |
|---|---|---|---|---|---|
| Me | CE | Me | CE | H | CE |
| Me | CE | Me | CE | CE | CE |
| Me | Me | Me | H | H | CE |
| Me | Me | Me | CE | H | H |
| Me | Me | Me | H | CE | CE |
| Me | Me | Me | CE | H | CE |
| Me | Me | Me | CE | CE | CE |
| Me | H | Me | H | Me | CE |
| Me | H | Me | CE | Me | CE |
| Me | CE | Me | CE | Me | CE |
| Me | Me | Me | Me | H | CE |
| Me | Me | Me | Me | CE | CE |
| Me | Me | Me | H | Me | CE |
| Me | Me | Me | CE | Me | CE |
| Me | Me | Me | Me | Me | CE |
| Et | H | H | H | H | CE |
| Et | CE | H | H | H | H |
| Et | H | H | H | CE | CE |
| Et | H | H | CE | H | CE |
| Et | CE | H | H | H | CE |
| Et | H | H | CE | CE | CE |
| Et | CE | H | H | CE | CE |
| Et | CE | H | CE | H | CE |
| Et | H | CE | CE | CE | CE |
| Et | CE | H | CE | CE | CE |
| Et | CE | CE | CE | CE | CE |
| Et | Et | H | H | H | CE |
| Et | Et | H | H | CE | CE |
| Et | Et | H | CE | H | CE |
| Et | Et | H | CE | CE | CE |
| Et | Et | CE | CE | CE | CE |
| Et | H | Et | H | H | CE |

**TABLE 1-3**

| X¹ | X² | X³ | X⁴ | X⁵ | X⁶ |
|---|---|---|---|---|---|
| Et | H | Et | CE | H | H |
| Et | H | Et | H | CE | CE |
| Et | H | Et | CE | H | CE |
| Et | CE | Et | CE | H | H |
| Et | H | Et | CE | CE | CE |
| Et | CE | Et | CE | H | CE |
| Et | CE | Et | CE | CE | CE |
| Et | Et | Et | H | H | CE |
| Et | Et | Et | CE | H | H |
| Et | Et | Et | H | CE | CE |
| Et | Et | Et | CE | H | CE |
| Et | Et | Et | CE | CE | CE |
| Et | H | Et | H | Et | CE |
| Et | H | Et | CE | Et | CE |
| Et | CE | Et | CE | Et | CE |
| Et | Et | Et | Et | H | CE |
| Et | Et | Et | Et | CE | CE |
| Et | Et | Et | H | Et | CE |
| Et | Et | Et | CE | Et | CE |
| Et | Et | Et | Et | Et | CE |
| Pr | H | H | H | H | CE |
| Pr | CE | H | H | H | H |
| Pr | H | H | H | CE | CE |
| Pr | H | H | CE | H | CE |
| Pr | CE | H | H | H | CE |
| Pr | H | H | CE | CE | CE |
| Pr | CE | H | H | CE | CE |
| Pr | CE | H | CE | H | CE |
| Pr | H | CE | CE | CE | CE |
| Pr | CE | H | CE | CE | CE |
| Pr | CE | CE | CE | CE | CE |
| Pr | Pr | H | H | H | CE |

**TABLE 1-4**

| X¹ | X² | X³ | X⁴ | X⁵ | X⁶ |
|---|---|---|---|---|---|
| Pr | Pr | H | H | CE | CE |
| Pr | Pr | H | CE | H | CE |
| Pr | Pr | H | CE | CE | CE |
| Pr | Pr | CE | CE | CE | CE |
| Pr | H | Pr | H | H | CE |
| Pr | H | Pr | CE | H | H |
| Pr | H | Pr | H | CE | CE |
| Pr | H | Pr | CE | H | CE |
| Pr | CE | Pr | CE | H | H |
| Pr | H | Pr | CE | CE | CE |
| Pr | CE | Pr | CE | H | CE |
| Pr | CE | Pr | CE | CE | CE |
| Pr | Pr | Pr | H | H | CE |
| Pr | Pr | Pr | CE | H | H |
| Pr | Pr | Pr | H | CE | CE |
| Pr | Pr | Pr | CE | H | CE |
| Pr | Pr | Pr | CE | CE | CE |
| Pr | H | Pr | H | Pr | CE |
| Pr | H | Pr | CE | Pr | CE |
| Pr | CE | Pr | CE | Pr | CE |
| Pr | Pr | Pr | Pr | H | CE |
| Pr | Pr | Pr | Pr | CE | CE |
| Pr | Pr | Pr | H | Pr | CE |
| Pr | Pr | Pr | CE | Pr | CE |
| Pr | Pr | Pr | Pr | Pr | CE |
| ME | H | H | H | H | CE |
| ME | CE | H | H | H | H |
| ME | H | H | H | CE | CE |
| ME | H | H | CE | H | CE |
| ME | CE | H | H | H | CE |
| ME | H | H | CE | CE | CE |
| ME | CE | H | H | CE | CE |

**TABLE 1-5**

| X¹ | X² | X³ | X⁴ | X⁵ | X⁶ |
|---|---|---|---|---|---|
| ME | CE | H | CE | H | CE |
| ME | H | CE | CE | CE | CE |
| ME | CE | H | CE | CE | CE |
| ME | CE | CE | CE | CE | CE |
| ME | ME | H | H | H | CE |
| ME | ME | H | H | CE | CE |
| ME | ME | H | CE | H | CE |
| ME | ME | H | CE | CE | CE |
| ME | ME | CE | CE | CE | CE |
| ME | H | ME | H | H | CE |
| ME | H | ME | CE | H | H |
| ME | H | ME | H | CE | CE |
| ME | H | ME | CE | H | CE |
| ME | CE | ME | CE | H | H |
| ME | H | ME | CE | CE | CE |
| ME | CE | ME | CE | H | CE |
| ME | CE | ME | CE | CE | CE |
| ME | ME | ME | H | H | CE |
| ME | ME | ME | CE | H | H |
| ME | ME | ME | H | CE | CE |
| ME | ME | ME | CE | H | CE |
| ME | ME | ME | CE | CE | CE |
| ME | H | ME | H | ME | CE |
| ME | H | ME | CE | ME | CE |
| ME | CE | ME | CE | ME | CE |
| ME | ME | ME | ME | H | CE |
| ME | ME | ME | ME | CE | CE |
| ME | ME | ME | H | ME | CE |
| ME | ME | ME | CE | ME | CE |
| ME | ME | ME | ME | ME | CE |
| Bu | H | H | H | H | CE |
| Bu | CE | H | H | H | H |

**TABLE 1-6**

| X¹ | X² | X³ | X⁴ | X⁵ | X⁶ |
|---|---|---|---|---|---|
| Bu | H | H | H | CE | CE |
| Bu | H | H | CE | H | CE |
| Bu | CE | H | H | H | CE |
| Bu | H | H | CE | CE | CE |
| Bu | CE | H | H | CE | CE |
| Bu | CE | H | CE | H | CE |
| Bu | H | CE | CE | CE | CE |
| Bu | CE | H | CE | CE | CE |
| Bu | CE | CE | CE | CE | CE |
| Bu | Bu | H | H | H | CE |
| Bu | Bu | H | H | CE | CE |
| Bu | Bu | H | CE | H | CE |
| Bu | Bu | H | CE | CE | CE |
| Bu | Bu | CE | CE | CE | CE |
| Bu | H | Bu | H | H | CE |
| Bu | H | Bu | CE | H | H |
| Bu | H | Bu | H | CE | CE |
| Bu | H | Bu | CE | H | CE |
| Bu | CE | Bu | CE | H | H |
| Bu | H | Bu | CE | CE | CE |
| Bu | CE | Bu | CE | H | CE |
| Bu | CE | Bu | CE | CE | CE |
| Bu | Bu | Bu | H | H | CE |
| Bu | Bu | Bu | CE | H | H |
| Bu | Bu | Bu | H | CE | CE |
| Bu | Bu | Bu | CE | H | CE |
| Bu | Bu | Bu | CE | CE | CE |
| Bu | H | Bu | H | Bu | CE |
| Bu | H | Bu | CE | Bu | CE |
| Bu | CE | Bu | CE | Bu | CE |
| Bu | Bu | Bu | Bu | H | CE |
| Bu | Bu | Bu | Bu | CE | CE |

**TABLE 1-7**

| X¹ | X² | X³ | X⁴ | X⁵ | X⁶ |
|---|---|---|---|---|---|
| Bu | Bu | Bu | H | Bu | CE |
| Bu | Bu | Bu | CE | Bu | CE |
| Bu | Bu | Bu | Bu | Bu | CE |
| IB | H | H | H | H | CE |
| IB | CE | H | H | H | H |
| IB | H | H | H | CE | CE |
| IB | H | H | CE | H | CE |
| IB | CE | H | H | H | CE |
| IB | H | H | CE | CE | CE |
| IB | CE | H | H | CE | CE |
| IB | CE | H | CE | H | CE |
| IB | H | CE | CE | CE | CE |
| IB | CE | H | CE | CE | CE |
| IB | CE | CE | CE | CE | CE |
| IB | IB | H | H | H | CE |
| IB | IB | H | H | CE | CE |
| IB | IB | H | CE | H | CE |
| IB | IB | H | CE | CE | CE |
| IB | IB | CE | CE | CE | CE |
| IB | H | IB | H | H | CE |
| IB | H | IB | CE | H | H |
| IB | H | IB | H | CE | CE |
| IB | H | IB | CE | H | CE |
| IB | CE | IB | CE | H | H |
| IB | H | IB | CE | CE | CE |
| IB | CE | IB | CE | H | CE |
| IB | CE | IB | CE | CE | CE |
| IB | IB | IB | H | H | CE |
| IB | IB | IB | CE | H | H |
| IB | IB | IB | H | CE | CE |
| IB | IB | IB | CE | H | CE |
| IB | IB | IB | CE | CE | CE |

**TABLE 1-8**

| X¹ | X² | X³ | X⁴ | X⁵ | X⁶ |
|---|---|---|---|---|---|
| IB | H | IB | H | IB | CE |
| IB | H | IB | CE | IB | CE |
| IB | CE | IB | CE | IB | CE |
| IB | IB | IB | IB | H | CE |
| IB | IB | IB | IB | CE | CE |
| IB | IB | IB | H | IB | CE |
| IB | IB | IB | CE | IB | CE |
| IB | IB | IB | IB | IB | CE |
| MP | H | H | H | H | CE |
| MP | CE | H | H | H | H |
| MP | H | H | H | CE | CE |
| MP | H | H | CE | H | CE |
| MP | CE | H | H | H | CE |
| MP | H | H | CE | CE | CE |
| MP | CE | H | H | CE | CE |
| MP | CE | H | CE | H | CE |
| MP | H | CE | CE | CE | CE |
| MP | CE | H | CE | CE | CE |
| MP | CE | CE | CE | CE | CE |
| MP | MP | H | H | H | CE |
| MP | MP | H | H | CE | CE |
| MP | MP | H | CE | H | CE |
| MP | MP | H | CE | CE | CE |
| MP | MP | CE | CE | CE | CE |
| MP | H | MP | H | H | CE |
| MP | H | MP | CE | H | H |
| MP | H | MP | CE | H | H |
| MP | H | MP | CE | H | H |
| MP | H | MP | H | CE | CE |
| MP | H | MP | CE | H | CE |
| MP | CE | MP | CE | H | H |
| MP | H | MP | CE | CE | CE |

**TABLE 1-9**

| X¹ | X² | X³ | X⁴ | X⁵ | X⁶ |
|---|---|---|---|---|---|
| MP | CE | MP | CE | H | CE |
| MP | CE | MP | CE | CE | CE |
| MP | MP | MP | H | H | CE |
| MP | MP | MP | CE | H | H |
| MP | MP | MP | H | CE | CE |
| MP | MP | MP | CE | H | CE |
| MP | MP | MP | CE | CE | CE |
| MP | MP | MP | H | MP | CE |
| MP | H | MP | CE | MP | CE |
| MP | CE | MP | CE | MP | CE |
| MP | MP | MP | MP | H | CE |
| MP | MP | MP | MP | CE | CE |
| MP | MP | MP | H | MP | CE |
| MP | MP | MP | CE | MP | CE |
| MP | MP | MP | MP | MP | CE |
| TB | H | H | H | H | CE |
| TB | CE | H | H | H | H |
| TB | H | H | H | CE | CE |
| TB | H | H | CE | H | CE |
| TB | CE | H | H | H | CE |
| TB | H | H | CE | CE | CE |
| TB | CE | H | H | CE | CE |
| TB | CE | H | CE | H | CE |
| TB | H | CE | CE | CE | CE |
| TB | CE | H | CE | CE | CE |
| TB | CE | CE | CE | CE | CE |
| TB | TB | H | H | H | CE |
| TB | TB | H | H | CE | CE |
| TB | TB | CE | CE | CE | CE |
| TB | H | TB | H | H | CE |
| TB | H | TB | CE | H | H |
| TB | H | TB | H | CE | CE |

**TABLE 1-10**

| X¹ | X² | X³ | X⁴ | X⁵ | X⁶ |
|---|---|---|---|---|---|
| TB | H | TB | CE | H | CE |
| TB | CE | TB | CE | H | H |
| TB | H | TB | CE | CE | CE |
| TB | CE | TB | CE | H | CE |
| TB | CE | TB | CE | CE | CE |
| TB | TB | TB | H | H | CE |
| TB | TB | TB | CE | H | H |
| TB | TB | TB | H | CE | CE |
| TB | TB | TB | CE | H | CE |
| TB | TB | TB | CE | CE | CE |
| TB | H | TB | H | TB | CE |
| TB | H | TB | CE | TB | CE |
| TB | CE | TB | CE | TB | CE |
| TB | TB | TB | TB | H | CE |
| TB | TB | TB | TB | CE | CE |
| TB | TB | TB | H | TB | CE |
| TB | TB | TB | CE | TB | CE |
| TB | TB | TB | TB | TB | CE |
| PE | H | H | H | H | CE |
| PE | CE | H | H | H | H |
| PE | H | H | H | CE | CE |
| PE | H | H | CE | H | CE |
| PE | CE | H | H | H | CE |
| PE | H | H | CE | CE | CE |
| PE | CE | H | H | CE | CE |
| PE | CE | H | CE | H | CE |
| PE | H | CE | CE | CE | CE |
| PE | CE | H | CE | CE | CE |
| PE | CE | CE | CE | CE | CE |
| PE | PE | H | H | H | CE |
| PE | PE | H | H | CE | CE |
| PE | PE | H | CE | H | CE |

**TABLE 1-11**

| X¹ | X² | X³ | X⁴ | X⁵ | X⁶ |
|---|---|---|---|---|---|
| PE | PE | H | CE | CE | CE |
| PE | PE | CE | CE | CE | CE |
| PE | H | PE | H | H | CE |
| PE | H | PE | CE | H | H |
| PE | H | PE | H | CE | CE |
| PE | H | PE | CE | H | CE |
| PE | CE | PE | CE | H | H |
| PE | H | PE | CE | CE | CE |
| PE | CE | PE | CE | H | CE |
| PE | CE | PE | CE | CE | CE |
| PE | PE | PE | H | H | CE |
| PE | PE | PE | CE | H | H |
| PE | PE | PE | H | CE | CE |
| PE | PE | PE | CE | H | CE |
| PE | PE | PE | CE | CE | CE |
| PE | H | PE | H | PE | CE |
| PE | H | PE | CE | PE | CE |
| PE | CE | PE | CE | PE | CE |
| PE | PE | PE | PE | H | CE |
| PE | PE | PE | PE | CE | CE |
| PE | PE | PE | H | PE | CE |
| PE | PE | PE | CE | PE | CE |
| PE | PE | PE | PE | PE | CE |
| HE | H | H | H | H | CE |
| HE | CE | H | H | H | H |
| HE | H | H | H | CE | CE |
| HE | H | H | CE | H | CE |
| HE | CE | H | H | H | CE |
| HE | H | H | CE | CE | CE |
| HE | CE | H | H | CE | CE |
| HE | CE | H | CE | H | CE |
| HE | H | CE | CE | CE | CE |

**TABLE 1-12**

| X¹ | X² | X³ | X⁴ | X⁵ | X⁶ |
|---|---|---|---|---|---|
| HE | CE | H | CE | CE | CE |
| HE | CE | CE | CE | CE | CE |
| HE | HE | H | H | H | CE |
| HE | HE | H | H | CE | CE |
| HE | HE | H | CE | H | CE |
| HE | HE | H | CE | CE | CE |
| HE | HE | CE | CE | CE | CE |
| HE | H | HE | H | H | CE |
| HE | H | HE | CE | H | H |
| HE | H | HE | H | CE | CE |
| HE | H | HE | CE | H | CE |
| HE | CE | HE | CE | H | H |
| HE | H | HE | CE | CE | CE |
| HE | CE | HE | CE | H | CE |
| HE | CE | HE | CE | CE | CE |
| HE | HE | HE | H | H | CE |
| HE | HE | HE | CE | H | H |
| HE | HE | HE | H | CE | CE |
| HE | HE | HE | CE | H | CE |
| HE | HE | HE | CE | CE | CE |
| HE | H | HE | H | HE | CE |
| HE | H | HE | CE | HE | CE |
| HE | CE | HE | CE | HE | CE |
| HE | HE | HE | HE | H | CE |
| HE | HE | HE | HE | CE | CE |
| HE | HE | HE | H | HE | CE |
| HE | HE | HE | CE | HE | CE |
| HE | HE | HE | HE | HE | CE |
| EH | H | H | H | H | CE |
| EH | CE | H | H | H | H |
| EH | H | H | H | CE | CE |
| EH | H | H | CE | H | CE |

**TABLE 1-13**

| X¹ | X² | X³ | X⁴ | X⁵ | X⁶ |
|---|---|---|---|---|---|
| EH | CE | H | H | H | CE |
| EH | H | H | CE | CE | CE |
| EH | CE | H | H | CE | CE |
| EH | CE | H | CE | H | CE |
| EH | H | CE | CE | CE | CE |
| EH | CE | H | CE | CE | CE |
| EH | CE | CE | CE | CE | CE |
| EH | EH | H | H | H | CE |
| EH | EH | H | H | CE | CE |
| EH | EH | H | CE | H | CE |
| EH | EH | H | CE | CE | CE |
| EH | EH | CE | CE | CE | CE |
| EH | H | EH | H | H | CE |
| EH | H | EH | CE | H | H |
| EH | H | EH | H | CE | CE |
| EH | H | EH | CE | H | CE |
| EH | CE | EH | CE | H | H |
| EH | H | EH | CE | CE | CE |
| EH | CE | EH | CE | H | CE |
| EH | CE | EH | CE | CE | CE |
| EH | EH | EH | H | H | CE |
| EH | EH | EH | CE | H | H |
| EH | EH | EH | H | CE | CE |
| EH | EH | EH | CE | H | CE |
| EH | EH | EH | CE | CE | CE |
| EH | H | EH | H | EH | CE |
| EH | H | EH | CE | EH | CE |
| EH | CE | EH | CE | EH | CE |
| EH | EH | EH | EH | H | CE |
| EH | EH | EH | EH | CE | CE |
| EH | EH | EH | H | EH | CE |
| EH | EH | EH | CE | EH | CE |

**TABLE 1-14**

| X¹ | X² | X³ | X⁴ | X⁵ | X⁶ |
|---|---|---|---|---|---|
| EH | EH | EH | EH | EH | CE |
| Oc | H | H | H | H | CE |
| Oc | CE | H | H | H | H |
| Oc | H | H | H | CE | CE |
| Oc | H | H | CE | H | CE |
| Oc | CE | H | H | H | CE |
| Oc | H | H | CE | CE | CE |
| Oc | CE | H | H | CE | CE |
| Oc | CE | H | CE | H | CE |
| Oc | H | CE | CE | CE | CE |
| Oc | CE | H | CE | CE | CE |
| Oc | CE | CE | CE | CE | CE |
| Oc | Oc | H | H | H | CE |
| Oc | Oc | H | H | CE | CE |
| Oc | Oc | H | CE | H | CE |
| Oc | Oc | H | CE | CE | CE |
| Oc | Oc | CE | CE | CE | CE |
| Oc | H | Oc | H | H | CE |
| Oc | H | Oc | CE | H | H |
| Oc | H | Oc | H | CE | CE |
| Oc | H | Oc | CE | H | CE |
| Oc | CE | Oc | CE | H | H |
| Oc | H | Oc | CE | CE | CE |
| Oc | CE | Oc | CE | H | CE |
| Oc | CE | Oc | CE | CE | CE |
| Oc | Oc | Oc | H | H | CE |
| Oc | Oc | Oc | CE | H | H |
| Oc | Oc | Oc | H | CE | CE |
| Oc | Oc | Oc | CE | H | CE |
| Oc | Oc | Oc | CE | CE | CE |
| Oc | H | Oc | H | Oc | CE |
| Oc | H | Oc | CE | Oc | CE |

**TABLE 1-15**

| X¹ | X² | X³ | X⁴ | X⁵ | X⁶ |
|---|---|---|---|---|---|
| Oc | CE | Oc | CE | Oc | CE |
| Oc | Oc | Oc | Oc | H | CE |
| Oc | Oc | Oc | Oc | CE | CE |
| Oc | Oc | Oc | H | Oc | CE |
| Oc | Oc | Oc | CE | Oc | CE |
| Oc | Oc | Oc | Oc | Oc | CE |
| AL | H | H | H | H | CE |
| AL | CE | H | H | H | H |
| AL | H | H | H | CE | CE |
| AL | H | H | CE | H | CE |
| AL | CE | H | H | H | CE |
| AL | H | H | CE | CE | CE |
| AL | CE | H | H | CE | CE |
| AL | CE | H | CE | H | CE |
| AL | H | CE | CE | CE | CE |
| AL | CE | H | CE | CE | CE |
| AL | CE | CE | CE | CE | CE |
| AL | AL | H | H | H | CE |
| AL | AL | H | H | CE | CE |
| AL | AL | H | CE | H | CE |
| AL | AL | H | CE | CE | CE |
| AL | AL | CE | CE | CE | CE |
| AL | H | AL | H | H | CE |
| AL | H | AL | CE | H | H |
| AL | H | AL | H | CE | CE |
| AL | H | AL | CE | H | CE |
| AL | CE | AL | CE | H | H |
| AL | H | AL | CE | CE | CE |
| AL | CE | AL | CE | H | CE |
| AL | CE | AL | CE | CE | CE |
| AL | AL | AL | H | H | CE |
| AL | AL | AL | CE | H | H |

**TABLE 1-16**

| X¹ | X² | X³ | X⁴ | X⁵ | X⁶ |
|---|---|---|---|---|---|
| AL | AL | AL | H | CE | CE |
| AL | AL | AL | CE | H | CE |
| AL | AL | AL | CE | CE | CE |
| AL | H | AL | H | AL | CE |
| AL | H | AL | CE | AL | CE |
| AL | CE | AL | CE | AL | CE |
| AL | AL | AL | AL | H | CE |
| AL | AL | AL | AL | CE | CE |
| AL | AL | AL | H | AL | CE |
| AL | AL | AL | CE | AL | CE |
| AL | AL | AL | AL | AL | CE |
| ST | H | H | H | H | CE |
| ST | CE | H | H | H | H |
| ST | H | H | H | CE | CE |
| ST | H | H | CE | H | CE |
| ST | CE | H | H | H | CE |
| ST | H | H | CE | CE | CE |
| ST | CE | H | H | CE | CE |
| ST | CE | H | CE | H | CE |
| ST | H | CE | CE | CE | CE |
| ST | CE | H | CE | CE | CE |
| ST | CE | CE | CE | CE | CE |
| ST | ST | H | H | H | CE |
| ST | ST | H | H | CE | CE |
| ST | ST | H | CE | H | CE |
| ST | ST | H | CE | CE | CE |
| ST | ST | CE | CE | CE | CE |
| ST | H | ST | H | H | CE |
| ST | H | ST | CE | H | H |
| ST | H | ST | H | CE | CE |
| ST | H | ST | CE | H | CE |
| ST | CE | ST | CE | H | H |

**TABLE 1-17**

| X¹ | X² | X³ | X⁴ | X⁵ | X⁶ |
|---|---|---|---|---|---|
| ST | H | ST | CE | CE | CE |
| ST | CE | ST | CE | H | CE |
| ST | CE | ST | CE | CE | CE |
| ST | ST | ST | H | H | CE |
| ST | ST | ST | CE | H | H |
| ST | ST | ST | H | CE | CE |
| ST | ST | ST | CE | H | CE |
| ST | ST | ST | CE | CE | CE |
| ST | H | ST | H | ST | CE |
| ST | H | ST | CE | ST | CE |
| ST | CE | ST | CE | ST | CE |
| ST | ST | ST | ST | H | CE |
| ST | ST | ST | ST | CE | CE |
| ST | ST | ST | H | ST | CE |
| ST | ST | ST | CE | ST | CE |
| ST | ST | ST | ST | ST | CE |
| Cy | H | H | H | H | CE |
| Cy | CE | H | H | H | H |
| Cy | H | H | H | CE | CE |
| Cy | H | H | CE | H | CE |
| Cy | CE | H | H | H | CE |
| Cy | H | H | CE | CE | CE |
| Cy | CE | H | H | CE | CE |
| Cy | CE | H | CE | H | CE |
| Cy | H | CE | CE | CE | CE |
| Cy | CE | H | CE | CE | CE |
| Cy | CE | CE | CE | CE | CE |
| Cy | H | Cy | H | H | CE |
| Cy | H | Cy | CE | H | H |
| Cy | H | Cy | H | CE | CE |
| Cy | H | Cy | CE | H | CE |
| Cy | CE | Cy | CE | H | H |

**TABLE 1-18**

| X¹ | X² | X³ | X⁴ | X⁵ | X⁶ |
|---|---|---|---|---|---|
| Cy | H | Cy | CE | CE | CE |
| Cy | CE | Cy | CE | H | CE |
| Cy | CE | Cy | CE | CE | CE |
| Cy | H | Cy | H | Cy | CE |
| Cy | H | Cy | CE | Cy | CE |
| Cy | CE | Cy | CE | Cy | CE |
| CM | H | H | H | H | CE |
| CM | CE | H | H | H | H |
| CM | H | H | H | CE | CE |
| CM | H | H | CE | H | CE |
| CM | H | H | CE | CE | CE |
| CM | CE | H | H | H | CE |
| CM | CE | H | H | CE | CE |
| CM | CE | H | CE | H | CE |
| CM | H | CE | CE | CE | CE |
| CM | CE | H | CE | CE | CE |
| CM | CE | CE | CE | CE | CE |
| CM | H | CM | H | H | CE |
| CM | H | CM | CE | H | H |
| CM | H | CM | H | CE | CE |
| CM | H | CM | CE | H | CE |
| CM | CE | CM | CE | H | H |
| CM | H | CM | CE | CE | CE |
| CM | CE | CM | CE | H | CE |
| CM | CE | CM | CE | CE | CE |
| CM | H | CM | H | CM | CE |
| CM | H | CM | CE | CM | CE |
| CM | CE | CM | CE | CM | CE |
| Ph | H | H | H | H | CE |
| Ph | CE | H | H | H | H |
| Ph | H | H | H | CE | CE |
| Ph | H | H | CE | H | CE |

**TABLE 1-19**

| X¹ | X² | X³ | X⁴ | X⁵ | X⁶ |
|---|---|---|---|---|---|
| Ph | CE | H | H | H | CE |
| Ph | H | H | CE | CE | CE |
| Ph | CE | H | H | CE | CE |
| Ph | CE | H | CE | H | CE |
| Ph | H | CE | CE | CE | CE |
| Ph | CE | H | CE | CE | CE |
| Ph | CE | CE | CE | CE | CE |
| Ph | H | Ph | H | H | CE |
| Ph | H | Ph | CE | H | H |
| Ph | H | Ph | H | CE | CE |
| Ph | H | Ph | CE | H | CE |
| Ph | CE | Ph | CE | H | H |
| Ph | H | Ph | CE | CE | CE |
| Ph | CE | Ph | CE | H | CE |
| Ph | CE | Ph | CE | CE | CE |
| Ph | H | Ph | H | Ph | CE |
| Ph | H | Ph | CE | Ph | CE |
| Ph | CE | Ph | CE | Ph | CE |
| Bz | H | H | H | H | CE |
| Bz | CE | H | H | H | H |
| Bz | H | H | H | CE | CE |
| Bz | H | H | CE | H | CE |
| Bz | CE | H | H | H | CE |
| Bz | H | H | CE | CE | CE |
| Bz | CE | H | H | CE | CE |
| Bz | CE | H | CE | H | CE |
| Bz | H | CE | CE | CE | CE |
| Bz | CE | H | CE | CE | CE |
| Bz | CE | CE | CE | CE | CE |
| Bz | H | Bz | H | H | CE |
| Bz | H | Bz | CE | H | H |
| Bz | H | Bz | H | CE | CE |

**TABLE 1-20**

| X¹ | X² | X³ | X⁴ | X⁵ | X⁶ |
|---|---|---|---|---|---|
| Bz | H | Bz | CE | H | CE |
| Bz | CE | Bz | CE | H | H |
| Bz | H | Bz | CE | CE | CE |
| Bz | CE | Bz | CE | H | CE |
| Bz | CE | Bz | CE | CE | CE |
| Bz | H | Bz | H | Bz | CE |
| Bz | H | Bz | CE | Bz | CE |
| Bz | CE | Bz | CE | Bz | CE |
| Me | H | EH | H | H | CE |
| Me | H | EH | CE | H | H |
| Me | H | EH | H | CE | CE |
| Me | H | EH | CE | H | CE |
| Me | CE | EH | CE | H | H |
| Me | H | EH | CE | CE | CE |
| Me | CE | EH | CE | H | CE |
| Me | CE | EH | CE | CE | CE |
| Me | Me | EH | H | H | CE |
| Me | Me | EH | CE | H | H |
| Me | Me | EH | H | CE | CE |
| Me | Me | EH | CE | H | CE |
| Me | Me | EH | CE | CE | CE |
| Me | H | EH | H | Me | CE |
| Me | H | EH | CE | Me | CE |
| Me | CE | EH | CE | Me | CE |
| Me | Me | EH | H | Me | CE |
| Me | Me | EH | CE | Me | CE |
| Me | Me | Me | Me | EH | CE |
| Me | H | Cy | H | H | CE |
| Me | H | Cy | CE | H | H |
| Me | H | Cy | H | CE | CE |
| Me | H | Cy | CE | H | CE |
| Me | CE | Cy | CE | H | H |

**TABLE 1-21**

| X¹ | X² | X³ | X⁴ | X⁵ | X⁶ |
|---|---|---|---|---|---|
| Me | H | Cy | CE | CE | CE |
| Me | CE | Cy | CE | H | CE |
| Me | CE | Cy | CE | CE | CE |
| Me | Me | Cy | H | H | CE |
| Me | Me | Cy | CE | H | H |
| Me | Me | Cy | H | CE | CE |
| Me | Me | Cy | CE | H | CE |
| Me | Me | Cy | CE | CE | CE |
| Me | H | Cy | H | Me | CE |
| Me | H | Cy | CE | Me | CE |
| Me | CE | Cy | CE | Me | CE |
| Me | Me | Cy | H | Me | CE |
| Me | Me | Cy | CE | Me | CE |
| Me | Me | Me | Me | Cy | CE |
| Me | H | Bz | H | H | CE |
| Me | H | Bz | CE | H | H |
| Me | H | Bz | H | CE | CE |
| Me | H | Bz | CE | H | CE |
| Me | CE | Bz | CE | H | H |
| Me | H | Bz | CE | CE | CE |
| Me | CE | Bz | CE | H | CE |
| Me | CE | Bz | CE | CE | CE |
| Me | Me | Bz | H | H | CE |
| Me | Me | Bz | CE | H | H |
| Me | Me | Bz | H | CE | CE |
| Me | Me | Bz | CE | H | CE |
| Me | Me | Bz | CE | CE | CE |
| Me | H | Bz | H | Me | CE |
| Me | H | Bz | CE | Me | CE |
| Me | CE | Bz | CE | Me | CE |
| Me | Me | Bz | H | Me | CE |
| Me | Me | Bz | CE | Me | CE |

**TABLE 1-22**

| X¹ | X² | X³ | X⁴ | X⁵ | X⁶ |
|---|---|---|---|---|---|
| Me | Me | Me | Me | Bz | CE |
| Et | H | EH | H | H | CE |
| Et | H | EH | CE | H | H |
| Et | H | EH | H | CE | CE |
| Et | H | EH | CE | H | CE |
| Et | CE | EH | CE | H | H |
| Et | H | EH | CE | CE | CE |
| Et | CE | EH | CE | H | CE |
| Et | CE | EH | CE | CE | CE |
| Et | Et | EH | H | H | CE |
| Et | Et | EH | CE | H | H |
| Et | Et | EH | H | CE | CE |
| Et | Et | EH | CE | H | CE |
| Et | Et | EH | CE | CE | CE |
| Et | H | EH | H | Et | CE |
| Et | H | EH | CE | Et | CE |
| Et | CE | EH | CE | Et | CE |
| Et | Et | EH | H | Et | CE |
| Et | Et | EH | CE | Et | CE |
| Et | Et | Et | Et | EH | CE |
| Et | H | Cy | H | H | CE |
| Et | H | Cy | CE | H | H |
| Et | H | Cy | H | CE | CE |
| Et | H | Cy | CE | H | CE |
| Et | CE | Cy | CE | H | H |
| Et | H | Cy | CE | CE | CE |
| Et | CE | Cy | CE | H | CE |
| Et | CE | Cy | CE | CE | CE |
| Et | Et | Cy | H | H | CE |
| Et | Et | Cy | CE | H | H |
| Et | Et | Cy | H | CE | CE |
| Et | Et | Cy | CE | H | CE |

**TABLE 1-23**

| X¹ | X² | X³ | X⁴ | X⁵ | X⁶ |
|---|---|---|---|---|---|
| Et | Et | Cy | CE | CE | CE |
| Et | H | Cy | H | Et | CE |
| Et | H | Cy | CE | Et | CE |
| Et | CE | Cy | CE | Et | CE |
| Et | Et | Cy | H | Et | CE |
| Et | Et | Cy | CE | Et | CE |
| Et | Et | Et | Et | Cy | CE |
| Et | H | Bz | H | H | CE |
| Et | H | Bz | CE | H | H |
| Et | H | Bz | H | CE | CE |
| Et | H | Bz | CE | H | CE |
| Et | CE | Bz | CE | H | H |
| Et | H | Bz | CE | CE | CE |
| Et | CE | Bz | CE | H | CE |
| Et | CE | Bz | CE | CE | CE |
| Et | Et | Bz | H | H | CE |
| Et | Et | Bz | CE | H | H |
| Et | Et | Bz | H | CE | CE |
| Et | Et | Bz | CE | H | CE |
| Et | Et | Bz | CE | CE | CE |
| Et | H | Bz | H | Et | CE |
| Et | H | Bz | CE | Et | CE |
| Et | CE | Bz | CE | Et | CE |
| Et | Et | Bz | H | Et | CE |
| Et | Et | Bz | CE | Et | CE |
| Et | Et | Et | Et | Bz | CE |
| ME | H | EH | H | H | CE |
| ME | H | EH | CE | H | H |
| ME | H | EH | H | CE | CE |
| ME | H | EH | CE | H | CE |
| ME | CE | EH | CE | H | H |
| ME | H | EH | CE | CE | CE |

**TABLE 1-24**

| X¹ | X² | X³ | X⁴ | X⁵ | X⁶ |
|---|---|---|---|---|---|
| ME | CE | EH | CE | H | CE |
| ME | CE | EH | CE | CE | CE |
| ME | H | EH | H | ME | CE |
| ME | H | EH | CE | ME | CE |
| ME | CE | EH | CE | ME | CE |
| ME | H | Cy | H | H | CE |
| ME | H | Cy | CE | H | H |
| ME | H | Cy | H | CE | CE |
| ME | H | Cy | CE | H | CE |
| ME | CE | Cy | CE | H | H |
| ME | H | Cy | CE | CE | CE |
| ME | CE | Cy | CE | H | CE |
| ME | CE | Cy | CE | CE | CE |
| ME | H | Cy | H | ME | CE |
| ME | H | Cy | CE | ME | CE |
| ME | CE | Cy | CE | ME | CE |
| ME | H | Bz | H | H | CE |
| ME | H | Bz | CE | H | H |
| ME | H | Bz | H | CE | CE |
| ME | H | Bz | CE | H | CE |
| ME | CE | Bz | CE | H | H |
| ME | H | Bz | CE | CE | CE |
| ME | CE | Bz | CE | H | CE |
| ME | CE | Bz | CE | CE | CE |
| ME | H | Bz | H | ME | CE |
| ME | H | Bz | CE | ME | CE |
| ME | CE | Bz | CE | ME | CE |
| Bu | H | EH | H | H | CE |
| Bu | H | EH | CE | H | H |
| Bu | H | EH | H | CE | CE |
| Bu | H | EH | CE | H | CE |
| Bu | CE | EH | CE | H | H |

**TABLE 1-25**

| X¹ | X² | X³ | X⁴ | X⁵ | X⁶ |
|---|---|---|---|---|---|
| Bu | H | EH | CE | CE | CE |
| Bu | CE | EH | CE | H | CE |
| Bu | CE | EH | CE | CE | CE |
| Bu | Bu | EH | H | H | CE |
| Bu | Bu | EH | CE | H | H |
| Bu | Bu | EH | H | CE | CE |
| Bu | Bu | EH | CE | H | CE |
| Bu | Bu | EH | CE | CE | CE |
| Bu | H | EH | H | Bu | CE |
| Bu | H | EH | CE | Bu | CE |
| Bu | CE | EH | CE | Bu | CE |
| Bu | Bu | EH | H | Bu | CE |
| Bu | Bu | EH | CE | Bu | CE |
| Bu | Bu | Bu | Bu | EH | CE |
| Bu | H | Cy | H | H | CE |
| Bu | H | Cy | CE | H | H |
| Bu | H | Cy | H | CE | CE |
| Bu | H | Cy | CE | H | CE |
| Bu | CE | Cy | CE | H | H |
| Bu | H | Cy | CE | CE | CE |
| Bu | CE | Cy | CE | H | CE |
| Bu | CE | Cy | CE | CE | CE |
| Bu | Bu | Cy | H | H | CE |
| Bu | Bu | Cy | CE | H | H |
| Bu | Bu | Cy | H | CE | CE |
| Bu | Bu | Cy | CE | H | CE |
| Bu | Bu | Cy | CE | CE | CE |
| Bu | H | Cy | H | Bu | CE |
| Bu | H | Cy | CE | Bu | CE |
| Bu | CE | Cy | CE | Bu | CE |
| Bu | Bu | Cy | H | Bu | CE |
| Bu | Bu | Cy | CE | Bu | CE |

**TABLE 1-26**

| X¹ | X² | X³ | X⁴ | X⁵ | X⁶ |
|---|---|---|---|---|---|
| Bu | Bu | Bu | Bu | Cy | CE |
| Bu | H | Bz | H | H | CE |
| Bu | H | Bz | CE | H | H |
| Bu | H | Bz | H | CE | CE |
| Bu | H | Bz | CE | H | CE |
| Bu | CE | Bz | CE | H | H |
| Bu | H | Bz | CE | CE | CE |
| Bu | CE | Bz | CE | H | CE |
| Bu | CE | Bz | CE | CE | CE |
| Bu | Bu | Bz | H | H | CE |
| Bu | Bu | Bz | CE | H | H |
| Bu | Bu | Bz | H | CE | CE |
| Bu | Bu | Bz | CE | H | CE |
| Bu | Bu | Bz | CE | CE | CE |
| Bu | H | Bz | H | Bu | CE |
| Bu | H | Bz | CE | Bu | CE |
| Bu | CE | Bz | CE | Bu | CE |
| Bu | Bu | Bz | H | Bu | CE |
| Bu | Bu | Bz | CE | Bu | CE |
| Bu | Bu | Bu | Bu | Bz | CE |
| AL | H | EH | H | H | CE |
| AL | H | EH | CE | H | H |
| AL | H | EH | H | CE | CE |
| AL | H | EH | CE | H | CE |
| AL | CE | EH | CE | H | H |
| AL | H | EH | CE | CE | CE |
| AL | CE | EH | CE | H | CE |
| AL | CE | EH | CE | CE | CE |
| AL | AL | EH | H | H | CE |
| AL | AL | EH | CE | H | H |
| AL | AL | EH | H | CE | CE |
| AL | AL | EH | CE | H | CE |

**TABLE 1-27**

| X¹ | X² | X³ | X⁴ | X⁵ | X⁶ |
|---|---|---|---|---|---|
| AL | AL | EH | CE | CE | CE |
| AL | H | EH | H | AL | CE |
| AL | H | EH | CE | AL | CE |
| AL | CE | EH | CE | AL | CE |
| AL | AL | EH | H | AL | CE |
| AL | AL | EH | CE | AL | CE |
| AL | AL | AL | AL | EH | CE |
| AL | H | Cy | H | H | CE |
| AL | H | Cy | CE | H | H |
| AL | H | Cy | H | CE | CE |
| AL | H | Cy | CE | H | CE |
| AL | CE | Cy | CE | H | H |
| AL | H | Cy | CE | CE | CE |
| AL | CE | Cy | CE | H | CE |
| AL | CE | Cy | CE | CE | CE |
| AL | AL | Cy | H | H | CE |
| AL | AL | Cy | CE | H | H |
| AL | AL | Cy | H | CE | CE |
| AL | AL | Cy | CE | H | CE |
| AL | AL | Cy | CE | CE | CE |
| AL | H | Cy | H | AL | CE |
| AL | H | Cy | CE | AL | CE |
| AL | CE | Cy | CE | AL | CE |
| AL | AL | Cy | H | AL | CE |
| AL | AL | Cy | CE | AL | CE |
| AL | AL | AL | AL | Cy | CE |
| AL | H | Bz | H | H | CE |
| AL | H | Bz | CE | H | H |
| AL | H | Bz | H | CE | CE |
| AL | H | Bz | CE | H | CE |
| AL | CE | Bz | CE | H | H |
| AL | H | Bz | CE | CE | CE |

**TABLE 1-28**

| X¹ | X² | X³ | X⁴ | X⁵ | X⁶ |
|---|---|---|---|---|---|
| AL | CE | Bz | CE | H | CE |
| AL | CE | Bz | CE | CE | CE |
| AL | AL | Bz | H | H | CE |
| AL | AL | Bz | CE | H | H |
| AL | AL | Bz | H | CE | CE |
| AL | AL | Bz | CE | H | CE |
| AL | AL | Bz | CE | CE | CE |
| AL | H | Bz | H | AL | CE |
| AL | H | Bz | CE | AL | CE |
| AL | CE | Bz | CE | AL | CE |
| AL | AL | Bz | H | AL | CE |
| AL | AL | Bz | CE | AL | CE |
| AL | AL | AL | AL | Bz | CE |
| -(CH₂)₄- | | H | H | H | CE |
| -(CH₂)₄- | | H | H | CE | CE |
| -(CH₂)₄- | | H | CE | H | CE |
| -(CH₂)₄- | | H | CE | CE | CE |
| -(CH₂)₄- | | CE | CE | CE | CE |
| -(CH₂)₄- | | -(CH₂)₄- | | H | CE |
| -(CH₂)₄- | | -(CH₂)₄- | | CE | CE |
| -(CH₂)₅- | | H | H | H | CE |
| -(CH₂)₅- | | H | H | CE | CE |
| -(CH₂)₅- | | H | CE | H | CE |
| -(CH₂)₅- | | H | CE | CE | CE |
| -(CH₂)₅- | | CE | CE | CE | CE |
| -(CH₂)₅- | | -(CH₂)₅- | | H | CE |
| -(CH₂)₅- | | -(CH₂)₅- | | CE | CE |
| -(CH₂)₄- | | EH | H | H | CE |
| -(CH₂)₄- | | EH | CE | H | H |
| -(CH₂)₄- | | EH | CE | H | CE |
| -(CH₂)₄- | | EH | H | CE | CE |
| -(CH₂)₄- | | EH | CE | CE | CE |

**TABLE 1-29**

| X¹ | X² | X³ | X⁴ | X⁵ | X⁶ |
|---|---|---|---|---|---|
| -(CH₂)₄- | | -(CH₂)₄- | | EH | CE |
| -(CH₂)₄- | | Cy | H | H | CE |
| -(CH₂)₄- | | Cy | CE | H | H |
| -(CH₂)₄- | | Cy | CE | H | CE |
| -(CH₂)₄- | | Cy | H | CE | CE |
| -(CH₂)₄- | | Cy | CE | CE | CE |
| -(CH₂)₄- | | -(CH₂)₄- | | Cy | CE |
| -(CH₂)₄- | | Bz | H | H | CE |
| -(CH₂)₄- | | Bz | CE | H | H |
| -(CH₂)₄- | | Bz | CE | H | CE |
| -(CH₂)₄- | | Bz | H | CE | CE |
| -(CH₂)₄- | | Bz | CE | CE | CE |
| -(CH₂)₄- | | -(CH₂)₄- | | Bz | CE |
| -(CH₂)₅- | | EH | H | H | CE |
| -(CH₂)₅- | | EH | CE | H | H |
| -(CH₂)₅- | | EH | CE | H | CE |
| -(CH₂)₅- | | EH | H | CE | CE |
| -(CH₂)₅- | | EH | CE | CE | CE |
| -(CH₂)₅- | | -(CH₂)₅- | | EH | CE |
| -(CH₂)₅- | | Cy | H | H | CE |
| -(CH₂)₅- | | Cy | CE | H | H |
| -(CH₂)₅- | | Cy | CE | H | CE |
| -(CH₂)₅- | | Cy | H | CE | CE |
| -(CH₂)₅- | | Cy | CE | CE | CE |
| -(CH₂)₅- | | -(CH₂)₅- | | Cy | CE |
| -(CH₂)₅- | | Bz | H | H | CE |
| -(CH₂)₅- | | Bz | CE | H | H |
| -(CH₂)₅- | | Bz | CE | H | CE |
| -(CH₂)₅- | | Bz | H | CE | CE |
| -(CH₂)₅- | | Bz | CE | CE | CE |
| -(CH₂)₅- | | -(CH₂)₅- | | Bz | CE |

The above-mentioned compound examples constitute a part of representative examples of the compounds of the present invention and the present invention should not be limited thereto.

The various cyanoethylmelamine derivatives of the present invention are novel substances which, as they are, are useful as intermediates for various fine chemicals and it is possible to be converted into polyfunctional compounds having various reactive functional groups by further carrying out reactions of hydrolysis, reduction, etc. These derivatives can be applied to polymer materials, etc. as novel polyfunctional reactive intermediates.

### BEST MODE FOR CARRYING OUT THE INVENTION

Hereafter, the present invention will be described in more detail by examples. However, the present invention should not be limited thereto.

In the examples, the starting materials in all examples were synthesized according to "s-triazines and derivatives." from the series "The Chemistry of Heterocyclic Compounds," E. M. Smolin and L. Rapoport, Interscience Publishers Inc., New York, 1959. Synthesis examples of representative raw materials are shown in the following Reference Examples.

### Reference Example 1

### (Synthesis of 2,4-diamino-6-chloro-1,3,5-triazine)

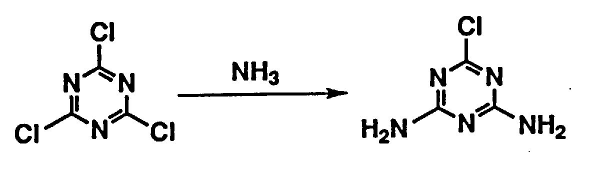

To a solution obtained by dissolving 184.5 g (1.0 mol) of cyanuric chloride in 800 ml of acetonitrile at room temperature and then cooling down to 0°C was dropwise added 303.7 g (5.0 mol) of an aqueous 28% ammonia solution over 2 hours with vigorous stirring by keeping the reaction temperature at 10°C or lower. After completion of the dropwise addition, the cooling was discontinued and the mixture was stirred at room temperature for 1 hour, followed by gradually warming the mixture up to 45°C and allowing the mixture to react 4 hours. After cooling, the product was filtered and washed with a large amount of water. The filtered product was dried at 50°C for 6 hours in vacuum to obtain 115 g (yield: 79%) of the titled compound.

### Reference Example 2

### (Synthesis of 4,6-diamino-2-normalbutylamino-1,3,5-triazine)

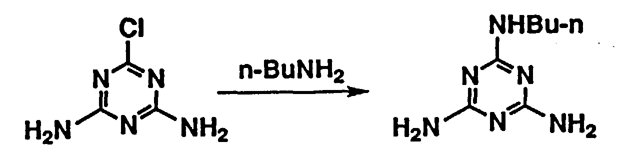

A mixed solution of 14.5 g (0.1 mol) of 2,4-diamino-6-chloro-1,3,5-triazine synthesized in Reference Example 1, 100 ml of water and 29.2 g (0.4 mol) of butylamine was warmed with stirring and allowed to react a reflux temperature for 6 hours. After cooling the reaction mixture, the product was filtered and washed sufficiently with a large amount of water and then washed with toluene. The filtrate was dried at 70°C for 6 hours in vacuum to obtain 17.5 g (yield: 96%) of the titled compound. Melting point: 167°C.

### Reference Example 3

### (Synthesis of 2-amino-4,6-bis(normalbutylamino)-1,3,5-triazine)

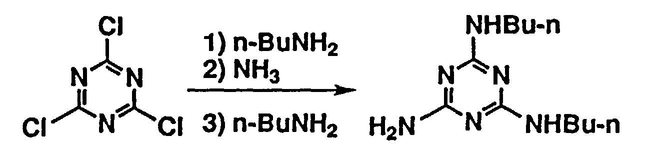

To a solution obtained by dissolving 18.5 g (0.1 mol) of cyanuric chloride in 150 ml of acetonitrile and cooling down to 0 °C was dropwise added to a solution of 7.3 g (0.1 mol) of butylamine in 20 ml of water over 1 hour with stirring by keeping the reaction temperature not to exceed 5°C. Further, while continuing the stirring, a solution of 10.0 g (0.1 mol) of potassium hydrogencarbonate in 100 ml of water was dropwise added at the same temperature and stirred for 3 hours. After confirming completion of the conversion to 2-butylamino-4,6-dichloro-1,3,5-triazine by high performance liquid chromatography, 24.3 g (0.4 mol) of an aqueous 28% ammonia solution was added to the resulting product and the mixture was warmed up to 50°C and reacted for 5 hours.

After cooling, the product was filtered and washed well with a large amount of water. The resultant crude product was suspended in 100 ml of water, to which was added 29.2 g (0.4 mol) of butylamine and the mixture was allowed to react for 6 hours by heating under reflux. After cooling, 200 ml of toluene was added and stirred vigorously, followed by separation of the aqueous layer. Further, the toluene layer was washed three times with 150 ml of water and then, toluene was distilled off from the organic layer by heating under reduced pressure to obtain 22.1 g (yield 93%) of the titled compound. Melting point: 73 °C.

### Reference Example 4

### (Synthesis of 2,4,6-tris(normalbutylamino)-1,3,5-triazine)

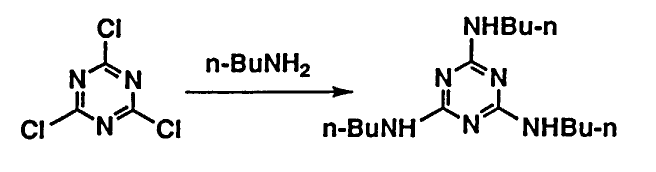

To a solution obtained by dissolving 18.5 g (0.1 mol) of cyanuric chloride in 150 ml of acetonitrile and cooling down to 0°C was dropwise added a solution of 14.6 g (0.2 mol) of butylamine in 20 ml of water over 1 hour with stirring by keeping the reaction temperature not to exceed 5°C . Futher, while continuing the stirring, a solution of 20.0 g (0.2 mol) of potassium hydrogencarbonate in 100 ml of water was dropwise added to the mixture at the same temperature.

Thereafter, the reaction temperature was elevated gradually and stirring was continued at 45°C for 8 hours. After confirming the completion of the conversion to 2,4-bis(butylamino)-6-chloro-1,3,5-triazine by high performance liquid chromatography, the reaction mixture was cooled and the product was separated by filtration. The filtration cake was washed well with a large amount of water, and the 2,4-bis(butylamino)-6-chloro-1,3,5-triazine was suspended in 100 ml of water. To the solution was added 29.2 g (0.4 mol) of butylamine and allowed to react by heating for 6 hours under reflux.

After cooling, 200 ml of toluene was added and the mixture was stirred vigorously, followed by separation of the aqueous layer. Further, the toluene layer was washed three times with 150 ml of water. Subsequently, toluene was distilled off from the organic layer by heating under reduced pressure to obtain 28.2 g (yield:96%) of the titled compound. Property: oily product.

### Reference Example 5

### (Synthesis of 4,6-diamino-2-normaloctylamino-1,3,5-triazine)

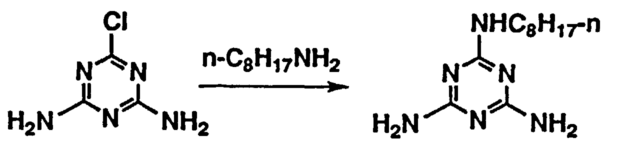

A mixed solution of 14.5 g (0.1 mol) of 2,4-diamino-6-chloro-1,3,5-triazine synthesized in Reference Example 1, 50 ml of water, 50 ml of 1,4-dioxane and 12.9 g (0.1 mol) of normaloctylamine was warmed with stirring and allowd to react finally at a reflux temperature for 2 hours. Subsequently, an aqueous solution of 4.0 g (0.1 mol) of sodium hydroxide in 20 ml of water was dropwise added to the mixture over 1 hour while continuing refluxing and the reaction was continued for additional 2 hours.

After cooling the reaction mixture, the product was extracted by addition of 100 ml of toluene and 100 ml of water, and the obtained organic layer was washed well with water. To the obtained toluene solution was concentrated under reduced pressure to obtain 23.1 g (yield: 97%) of the titled compound.
Melting point: 108°C.

### Reference Example 6

### (Synthesis of 4,6-diamino-2-piperidino-1,3,5-triazine)

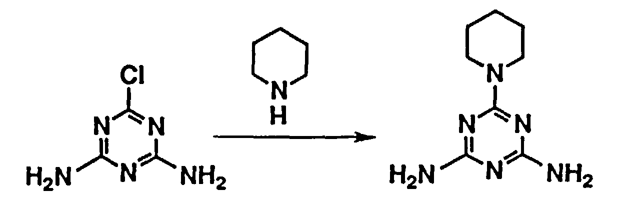

Synthesis was carried out in the same manner as in Reference Example 5. Obtained amount was 18.4 g (yield:95%). Melting point: 210°C.

Examples according to the methods of the present invention are cited below.

### Example 1

### (Synthesis of 2,4,6-tris(N-normalbutyl-N-cyanoethylamino)-1,3,5-triazine (Production method: A))

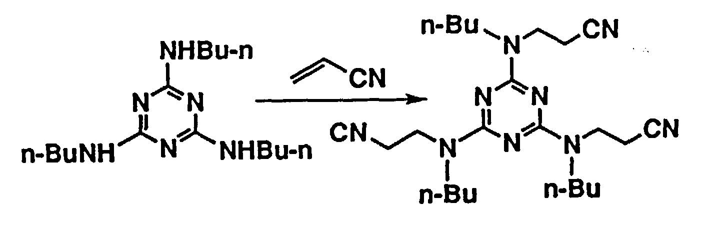

In a 200 ml reaction flask was charged a solution of 5.88 g (20 mmol) of 2,4,6-tris(normalbutylamino)-1,3,5-triazine in 50 ml of acetonitrile and 0.5 g of pulverized potassium hydroxide was added thereto and then the temperature was elevated to 70°C and the mixture was stirred for 30 minutes. Subsequently, 4.77 g (90 mmol) of acrylonitrile was dropwise added to the mixture over 1 hour by keeping the reaction temperature at 70°C . Thereafter, the stirring was carried out at the same temperature for 2 hours to complete the reaction.

After cooling the reaction mixture, the solvent was distilled off under reduced pressure and chloroform and water each in 200 ml were added to perform extraction operation. The obtained chloroform layer was serially washed with water, an aqueous 5% hydrochloric acid solution, water and saturated aqueous solution of sodium chloride. The solvent was distilled off from the organic layer under reduced pressure and the obtained viscous oily product purified by silica gel column chromatography (eluant; chloroform : ethyl acetate = 4:1) to obtain 5.71 g (isolation yield: 63%) of the intended compound as colorless crystal.
Melting point: 119.1°C.

### Example 2

### (Synthesis of 2-cyanoehtylamino-4,6-dipiperidino-1,3,5-triazine (Production method: B))

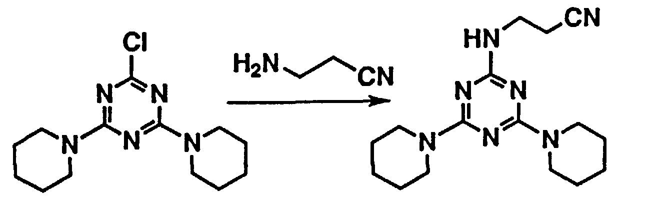

In a 200 ml-reaction flask were charged 2.82 g (10 mmol) of 2-chloro-4,6-dipiperidino-1,3,5-triazine, 30 ml of 1,4-dioxane and 30 ml of water, and the mixture was warmed to 40°C to obtain a slurry, to which was gradually added 1.40 g (20 mmol) of aminopropionitrile. Subsequently the temperature was elevated to reflux temperature and stirring was performed for 2 hours. Further, 10 ml of aqueous solution of 0.40 g (10 mmol) of sodium hydroxide was dropwise added over 1 hour and the reaction was carried out for additional 3 hours.

After cooling the homogenous solution after the completion of the reaction to room temperature, the solvent was distilled off under reduced pressure. To the residue was added chloroform and water each in 200 ml and extraction operation was conducted to obtain a chloroform layer, which was serially washed with water, an aqueous 5% hydrochloric acid solution, water and a saturated aqueous solution of sodium chloride. The solvent was distilled off from the organic layer under reduced pressure. The obtained residue was purified by silica gel column chromatography (eluant; chloroform : ethyl acetate = 4:1) to obtain 2.90 g (isolation yield: 92%) of the intended compound as colorless crystal. Melting point: 103.9°C.

### EXAMPLE 3

The following compounds were synthesized and isolated in a manner similar to those in the previous examples. Table 2 below shows a list of the synthesized compounds inclusive of the compounds of Examples 1 and 2 (Example 1: No. 11, Example 2: No. 21).

The compounds in the table show main products obtained by using as raw materials the compounds having H (hydrogen) instead of CE (cyanoethyl) group in Table 2 when the compounds were produced in accordance with the above-mentioned Example 1 (production method: A) or by using compounds with the chlorine atom on the triazine nucleus having been converted to a cyanoethylamino group when compounds were produced in accordance with Example 2 (production method: B).

In the case of compound No. 5 only, the second component was produced in a considerable amount and hence it was isolated and determined for its chemical structure.

In Table 2, abbreviations for substituents have the following meanings.

| | |
|---|---|
| Me: methyl group | Et: ethyl group |
| Pr: normalpropyl group | Bu: normalbutyl group |
| HE: normalhexyl group | EH: 2-ethylhexyl group |
| Oc: normaloctyl group | Cy: cyclohexyl group |
| CM: cyclohexylmethyl group | Bz: benzyl group |
| CE: cyanoethyl group | |

Further, the -(CH₂)₅- group indicates that the nitrogen atom on the triazine ring forms a piperidine ring containing 5 methylene groups.

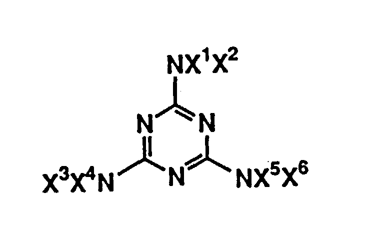

**TABLE 2**

| List of Synthesized Compounds | | | | | | |
|---|---|---|---|---|---|---|
| Compound No. | X¹ | X² | X³ | X⁴ | X⁵ | X⁶ |
| 1 | Me | CE | CE | CE | CE | CE |
| 2 | Me | CE | Me | CE | Me | CE |
| 3 | Et | CE | Et | CE | Et | CE |
| 4 | Et | Et | Et | Et | Et | CE |
| 5 | Pr | CE | H | CE | CE | CE |
| 6 | Pr | CE | CE | CE | CE | CE |
| 7 | Pr | Pr | CE | CE | CE | CE |
| 8 | Bu | CE | CE | CE | CE | CE |
| 9 | Bu | Bu | CE | CE | CE | CE |
| 10 | Bu | CE | Bu | CE | CE | CE |
| 11 | Bu | CE | Bu | CE | Bu | CE |
| 12 | Bu | Bu | H | H | H | CE |
| 13 | HE | CE | CE | CE | CE | CE |
| 14 | HE | CE | HE | CE | HE | CE |
| 15 | EH | CE | CE | CE | CE | CE |
| 16 | EH | CE | EH | CE | EH | CE |
| 17 | Cy | H | Cy | H | CE | CE |
| 18 | Cy | H | Cy | CE | CE | CE |
| 19 | -(CH₂)₅- | | CE | CE | CE | CE |
| 20 | -(CH₂)₅- | | -(CH₂)₅- | | H | CE |
| 21 | Bz | CE | Bz | CE | Bz | CE |
| 22 | Oc | CE | CE | CE | CE | CE |
| 23 | CM | H | CM | H | H | CE |

The synthesis methods (production methods: A and B) of the synthesized compounds, isolation yield (%) and physical data described as above are gathered in Table 3 below.

**Table 3-1**

| Data of Synthesized Compounds | | | | |
|---|---|---|---|---|
| Compound No. | Production method | Yield | Melting point °C | ¹H-NMR data (δ , ppm, CDCl₃) |
| 1 | A | 72% | 14.7 | 2.61(2H,t,J=6Hz), |
| | | | | 2.72(8H,t,J=6Hz), |
| | | | | 3.12(3H,s), |
| | | | | 3.82(2H,t,J=6Hz), |
| | | | | 3.86(8H,t,J=6Hz) |
| 2 | A | 66 | 104.9 | 2.67(6H,t,J=7Hz), |
| | | | | 3.15(9H,s), |
| | | | | 3.81(6H,t,J=7Hz) |
| 3 | A | 71 | 138.2 | 1.15(9H,t,J=7Hz), |
| | | | | 2.67(6H,t,J=7Hz), |
| | | | | 3.56(6H,q,J=7Hz), |
| | | | | 3.72(6H,t,J=7Hz) |
| 4 | A | 61 | oil | 1.14(15H,t,J=7Hz), |
| | | | | 2.69(2H,J=7Hz), |
| | | | | 3.49(8H,q,J=7Hz), |
| | | | | 3.58(2H,t,J=7Hz), |
| | | | | 3.71(2H,t,J=7Hz) |
| 5 | A | 22 | 110.0 | 0.91(3H,t,J=7Hz), |
| | | | | 1.61(2H,m), |
| | | | | 2.55-2.81(8H,m), |
| | | | | 3.50(2H,q,J=7Hz), |
| | | | | 3.57-3.91(8H,m), |
| | | | | 5.29(1H,t) |
| 6 | A | 41 | 133.0 | 0.93(3H,t,J=7Hz), |
| | | | | 1.62(2H,m), |
| | | | | 2.65(2H,t,J=6Hz), |
| | | | | 2.71(8H,t,J=6Hz), |
| | | | | 3.48(2H,t,J=7Hz), |
| | | | | 3.75(10H,m) |
| 7 | A | 59 | 115.8 | 0.90(6H,t,J=7Hz), |
| | | | | 1.57(4H,m), |
| | | | | 2.70(8H,t,J=6.5Hz), |
| | | | | 3.37(4H,t,J=7Hz), |
| | | | | 3.82(8H,t,J=6.5Hz) |

**Table 3-2**

| Data of Synthesized Compounds | | | | |
|---|---|---|---|---|
| Compound No. | Production method | Yield | Melting point °C | ¹H-NMR data (δ , ppm, CDCl₃) |
| 8 | A | 66% | 100.5 | 0.93(3H,t,J=7Hz), |
| | | | | 1.20-1.65(4H,m), |
| | | | | 2.63(2H,t,J=6Hz), |
| | | | | 2.69(8H,t,J=6Hz), |
| | | | | 3.56(2H,t,J=7Hz) |
| | | | | 3.81(2H,t,J=6Hz), |
| | | | | 3.84(8H,t,J=6Hz) |
| 9 | A | 78 | 100.4 | 0.93(6H,t,J=7Hz), |
| | | | | 1.19-1.65(8H,m), |
| | | | | 2.70(8H,t,J=6Hz) |
| | | | | 3.40(4H,t,J=7Hz), |
| | | | | 3.81(8H,t,J=6Hz) |
| 10 | A | 71 | 97.5 | 0.94(6H,t,J=7Hz), |
| | | | | 1.20-1.70(8H,m), |
| | | | | 1.64(4H,t,J=6Hz), |
| | | | | 1.72(4H,t,J=6Hz) |
| | | | | 3.58(4H,t,J=7Hz), |
| | | | | 3.75(4H,t,J=6Hz), |
| | | | | 3.86(4H,t,J=6Hz) |
| 11 | A | 63 | 119.1 | 0.94(9H,t,J=7Hz), |
| | | | | 1.20-1.75(12H,m), |
| | | | | 2.65(6H,t,J=6.5Hz), |
| | | | | 3.49(6H,t,J=7Hz), |
| | | | | 3.71(6H,t,J=6.6Hz) |
| 12 | B | 94 | 64.1 | 0.91(6H,t,J=7Hz), |
| | | | | 1.16-1.70(8H,m), |
| | | | | 2.62(2H,t,J=6.5Hz), |
| | | | | 3.39(4H,t,J=7Hz), |
| | | | | 3.48(2H,m), |
| | | | | 4.97(2H,broad s), |
| | | | | 5.96(1H,t) |
| 13 | A | 72 | 103.2 | 0.89(3H,t,J=6Hz), |
| | | | | 1.31-1.67(8H,m), |
| | | | | 2.65(2H,t,J=6.5Hz), |
| | | | | 2.71(8H,t,J=6.5Hz), |
| | | | | 3.50(2H,t,J=6Hz), |
| | | | | 3.85(10H,t,J=6.5Hz) |

**Table 3-3**

| Data of Synthesized Compounds | | | | |
|---|---|---|---|---|
| Compound No. | Production method | Yield | Melting point °C | ¹H-NMR data (δ , ppm, CDCl₃) |
| 14 | A | 62 | 56.5 | 0.87(9H,t,J=6Hz), |
| | | | | 1.10-1.83(24H,m), |
| | | | | 2.65(6H,t,J=6.5Hz), |
| | | | | 3.51(6H,t,J=6Hz), |
| | | | | 3.72(6H,t,J=6.5Hz) |
| 15 | A | 58% | 72.3 | 0.90(6H,t,J=6Hz), |
| | | | | 1.15-1.83(9H,m), |
| | | | | 2.65(2H,t,J=6Hz) |
| | | | | 2.71(8H,t,J=6Hz), |
| | | | | 3.44(2H,d,J=6Hz) |
| | | | | 3.76(2H,t,J=6Hz) |
| | | | | 3.86(8H,t,J=6Hz) |
| 16 | A | 67 | oil | 0.87(18H,t,J=6Hz), |
| | | | | 1.10-1.88(27H,m), |
| | | | | 2.65(6H,t,J=6.5Hz), |
| | | | | 3.46(6H,d,J=6Hz) |
| | | | | 3.74(6H,t,J=6.5Hz) |
| 17 | A | 31 | 128.0 | 0.85-2.13(20H,m), |
| | | | | 2.71(4H,t,J=6Hz), |
| | | | | 3.63(2H,m), |
| | | | | 3.78(4H,t,J=6Hz) |
| | | | | 4.83(2H,d,J=8Hz) |
| 18 | A | 18 | 171.5 | 1.02-2.15(20H,m), |
| | | | | 2.63(2H,t,J=6Hz), |
| | | | | 2.71(4H,t,J=6Hz), |
| | | | | 3.60(2H,m), |
| | | | | 3.70(2H,t,J=6Hz), |
| | | | | 3.79(4H,t,J=6Hz), |
| | | | | 4.76(1H,d,J=8Hz) |
| 19 | A | 73 | 116.2 | 1.41-1.77(6H,m), |
| | | | | 2.72(8H,t,J=6.5Hz), |
| | | | | 3.51-3.75(4H,m), |
| | | | | 3.85(8H,t,J=6.5Hz) |

**Table 3-4**

| Data of Synthesized Compounds | | | | |
|---|---|---|---|---|
| Compound No. | Production method | Yield | Melting point °C | ¹H-NMR data (δ , ppm, CDCl₃) |
| 20 | B | 92 | 103.9 | 1.40-1.73(12H,m), |
| | | | | 2.64(2H,t,J=6.5Hz), |
| | | | | 3.42-3.80(10H,m), |
| | | | | 4.95(1H,t,J=6Hz), |
| 21 | A | 71 | 130.5 | 2.45(6H,broad t), |
| | | | | 3.69(6H,broad t), |
| | | | | 4.80(6H,S), |
| | | | | 7.06-7.35(15H,m), |
| 22 | A | 68% | 99.6 | 0.87(3H,t,J=6Hz), |
| | | | | 1.15-1.70(12H,m), |
| | | | | 2.64(2H,t,J=6Hz), |
| | | | | 2.71(8H,d,J=6Hz) |
| | | | | 3.46(2H,t,J=6Hz), |
| | | | | 3.74(2H,t,J=6Hz), |
| | | | | 3.84(8H,t,J=6Hz) |
| 23 | B | 86 | | 0.70-1.95(22H,m), |
| | | | | 2.65(2H,t,J=6.5Hz), |
| | | | | 3.14(4H,t,J=6Hz), |
| | | | | 3.55(2H,q,J=6.5Hz), |
| | | | | 5.11(2H,broad t), |
| | | | | 5.71(1H,broad t) |

In Table 3, the production method A or B shows the production method A or the production method B in Examples 1 and 2, yield shows the isolation yield of the product to the raw material triazine derivative, melting point shows data meastured by using FP62 type automatic melting point measuring apparatus manufactured by METTLER, ¹H-NMR data saws data measured by using JNM-PMX60st type Proton NMR measuring apparatus (60 MHz) manufactured by Nihon Electron Co., Ltd.

### INDUSTRIAL APPLICABILITY

According to the method of the present invention, novel N-cyanoethylmelamine derivatives, which are useful compounds widely employed as intermediates for various fine chemicals such as various agricultural chemicals, medicines, dye stuffs, paints, etc. and also as intermediates for crosslinking agents for various resins can be produced easily and at high yields under relatively mild reaction conditions from N-substituted melamines and N-substituted triazines of the formula II or the formula III, respectively.

Further, novel N-cyanoethylmelamine derivatives obtained by this reaction are polyfunctional compounds which can be easily converted to derivatives such as polycarboxylic acids and polyvalent amines. From the viewpoint of physical properties, they are considerably improved in solubility in various organic solvents as compared with conventional melamines and they include many interesting compounds with respect to stability at high temperatures, melting point, boiling point, basicity, etc. so that they are considered novel compounds having extremely high industrial feasibility.

## Claims

1. A cyanoethylmelamine of the formula I: 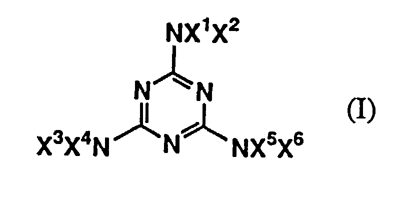 {wherein X¹, X², X³, X⁴, X⁵ and X⁶ independently represent respective substituents, 1 to 5 of which represent a cyanoethyl group and at least one remaining group represents a C₁₋₂₀ alkyl group, a C₂₋₂₀ alkenyl group (said alkyl or alkenyl group may optionally have an alicyclic group or a phenyl group in its structure), a C₅₋₆ cycloalkyl group or a phenyl group, or two of the substituent groups bonded to the same nitrogen atom together may form an alkylene chain having 2 to 7 carbon atoms, and the remaining substituent or substituents represents a hydrogen atom).

2. A cyanoethylmelamine derivatives as claimed in claim 1, wherein X¹, X², X³, X⁴, X⁵ and X⁶ independently represent respective substituents, 1 to 5 of which represent a cyanoethyl group, at least one remaining substituentre presents a C₁₋₁₀ alkyl group, a cyclohexylmethyl group, a cyclohexyl group or a benzyl group, or two of the above-mentioned substituents when bonded to the same nitrogen atom together may form an alkylene chain having 4 or 5 carbon atoms, and the remaining substituent or substituents represent a hydrogen atom.

3. A method for obtaining a cyanoethylmelamine derivative of the formula I (wherein the definitions of X¹, X², X³, X⁴, X⁵ and X⁶ are the same as in claim 1) described in claim 1 by reacting an N-substituted melamine derivative of the formula II: 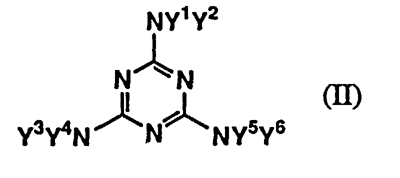 {wherein Y¹, Y², Y³, Y⁴, Y⁵ and Y⁶ independently represent respective substituents, 1 to 5 of which represent a C₁₋₂₀ alkyl group, a C₂₋₂₀ alkenyl group (said alkyl or alkenyl group may optionally have an alicyclic group or a phenyl group in its structure), a C₅₋₆ cycloalkyl group or a phenyl group, or two of the substituents bonded to the same nitrogen atom together may form an alkylene chain having 2 to 7 carbon atoms, and the remaining 5 to 1 substituents represent a hydrogen atom} with acrylonitrile.

4. A method as claimed in claim 3, wherein Y¹, Y², Y³, Y⁴, Y⁵ and Y⁶ independently represent respective substituents, 1 to 5 of which represent a C₁₋₁₀ alkyl group, a cyclohexylmethyl group, a cyclohexyl group or a benzyl group, or two of the above-mentioned substituents when bonded to the same nitrogen atom together may form an alkylene chain having 4 or 5 carbon atoms, and the remaining 5 to 1 substituents represent a hydrogen atom; and
in the formula I, X¹, X², X³, X⁴, X⁵ and X⁶ independently represent respective substituents, 1 to 5 of which represent a cyanoethyl group, at least one remaining substituent represents a C₁₋₁₀ alkyl group, a cyclohexylmethyl group, a cyclohexyl group or a benzyl group, or two of the above-mentioned substituents when bonded to the same nitrogen atom together may form an alkylene chain having 4 or 5 carbon atoms, and the remaining substituents represent a hydrogen atom.

5. A method for producing a cyanoethylmelamine derivative of the formula I (wherein the definitions of X¹, X², X³, X⁴, X⁵ and X⁶ are the same as in claim 1, provided that at least one of X¹ to X⁶ bonded to the nitrogen atoms to which the cyanoethyl group is bonded is a hydrogen atom) described in claim 1 by reacting an N-substituted triazine derivative of the formula III: 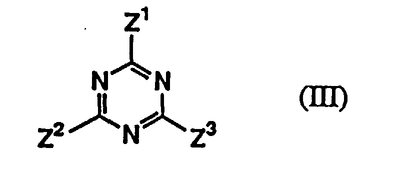 [wherein Z¹, Z² and Z³ independently represent respective substituents, 1 or 2 of which independently represent NX⁷X⁸ group (wherein X⁷ or X⁸ independently represents a C₁₋₂₀ alkyl group, a C₂₋₂₀ alkenyl group (said alkyl or alkenyl group may optionally have an alicyclic group or a phenyl group in its structure), a C₅₋₆ cycloalkyl group, a phenyl group or a hydrogen atom, or X⁷ and X⁸ together may form an alkylene group having 2 to 7 carbon atoms}, and the remaining 2 to 1 substituents represent a halogen atom] with aminopropionitrile.

6. A method as claimed in claim 5, wherein Z¹, Z² and Z³ independently represents respective substituents, 1 or 2 of which independently represent an NX⁷X⁸ group (wherein X⁷ or X⁸ independently represents a C₁₋₄ alkyl group, a cycloalkyl group, or a hydrogen atom, or X⁷ and X⁸ together may form an alkylene chain having 4 or 5 carbon atoms), and the 2 or 1 remaining substituents represent a chlorine atom, and
in the formula I, X¹, X², X³, X⁴, X⁵ and X⁶ independently represent respective substituents, one or two of substituted amino groups NX¹X², NX³X⁴ and NX⁵X⁶ independently represent NX⁷X⁸ group (wherein X⁷ or X⁸ independently represents a C₁₋₄ alkyl group, a cyclohexylmethyl group or a hydrogen atom, or X⁷ and X⁸ together may form an alkylene chain having 4 or 5 carbon atoms), and the remaining 2 or 1 substitueted amino groups represent a cyanoethylamino group.
